# EUROPEAN PATENT SPECIFICATION

(11) **EP 4 202 044 B1**
(45) Date of publication and mention of the grant of the patent: **14.10.2026**
(21) Application number: 22211393.8
(22) Date of filing: 05.12.2022
(51) Int. Cl.: C12N 9/80, C12P 35/06, C12P 17/06

(54) **POLYPEPTIDE HAVING CEPHALOSPORIN C ACYLASE ACTIVITY AND USE THEREOF**
POLYPEPTID MIT CEPHALOSPORIN C-ACYLASE-AKTIVITÄT UND VERWENDUNG DAVON
POLYPEPTIDE AYANT UNE ACTIVITÉ CÉPHALOSPORINE C ACYLASE ET SON UTILISATION

(30) Priority: 24.12.2021 KR 20210187761
(43) Date of publication of application: 28.06.2023
(73) Proprietor: Amicogen, Inc., Jinju-si, Gyeongsangnam-do 52840 (KR)
(72) Inventor: PIAO, Zhe, 52856 Gyeongsangnam-do (KR); WANG, Eun Seon, 52817 Gyeongsangnam-do (KR); LI, Hong Xian, 52860 Gyeongsangnam-do (KR); LEE, Mi Kyoung, 52834 Gyeongsangnam-do (KR); SON, Ah Reum, 52826 Gyeongsangnam-do (KR); LIM, Su Jin, 52838 Gyeongsangnam-do (KR)
(74) Representative: Grünecker Patent- und Rechtsanwälte PartG mbB

(56) References cited:
- EP-A1- 1 553 175
- WO-A1-2018/165881
- CN-A- 109 913 436
- CN-A- 111 172 142
- KR-A- 20140 094 150
- HARDIANTO DUDI ET AL: "Cephalosporin C Acylase from Microbes for One-step Enzymatic Transformation of Cephalosporin C to 7-Aminocephalosporanic Acid", JOURNAL OF PURE AND APPLIED MICROBIOLOGY, vol. 10, no. 4, 31 December 2016 (2016-12-31), IN, pages 2495 - 2499, XP93035626, ISSN: 0973-7510, Retrieved from the Internet <URL:https://www.microbiologyjournal.org/wp-content/uploads/2018/03/JPAM.10.4.03.pdf> DOI: 10.22207/JPAM.10.4.03

## Description

### BACKGROUND OF THE INVENTION

### 1. Field of the Invention

The present description relates to a polypeptide having cephalosporin C(CPC) acylase activity and a use thereof and, more specifically, to a mutant CPC acylase that has a point mutation introduced thereinto and thus exhibits improved enzymatic activity and/or stability, and a use thereof for producing 7-aminocephalosporanic acid (7-ACA).

### 2. Description of the Related Art

Cephalosporin C (hereinafter, referred to as "CPC"), which is a family of betalactam antibiotics, is produced by microorganisms such as *Acremonium chrysogenum.* CPC exhibits antibiotic activity against Gram-negative bacteria by inhibiting cell wall synthesis in Gram-negative bacteria, but due to its very weak activity, finds a main application in preparing raw materials for semi-synthetic cephalosporin antibiotics (hereinafter referred to as "cephalosporins"). Among others, 7-aminocephalosporanic acid (hereinafter, referred to as "7-ACA"), obtained by removing the D-alpha-aminoadipoyl side chain from CPC, is used as a raw material for most cephalosporins accounting for more than 40 % of the global antibiotic market.

Conventionally, there are chemical and enzymatic processes for preparing 7-ACA from CPC. Since the chemical processes require complex reaction conditions and high environmental treatment costs, the production of 7-ACA has recently tended to resort to environmentally friendly enzymatic processes.

CN 111 172 142 A relates to a cephalosporin C acylase mutant with high thermal stability. CN 109 913 436 A relates to a ephalosporin C acylase mutant and a preparation method thereof. EP 1 553 175 A1 relates to cephalosporin C acylase enzymes obtained by site-directed, random or "site-saturation" mutagenesis of a native sequence optimised for expression in *E*. *coli.* KR 2014 0094150 A relates to a mutant enzyme for producing a 7-ACA. WO 2018/165881 A1 relates to cephalosporin C acylase mutants constructed by means of a point mutation method. Hardianto Dudi et al. (JOURNAL OF PURE AND APPLIED MICROBIOLOGY, vol. 10, no. 4, 31 December 2016, pages 2495-2499) relates to cephalosporin C acylase from microbes for one-step enzymatic transformation of cephalosporin C to 7-aminocephalosporanic acid.

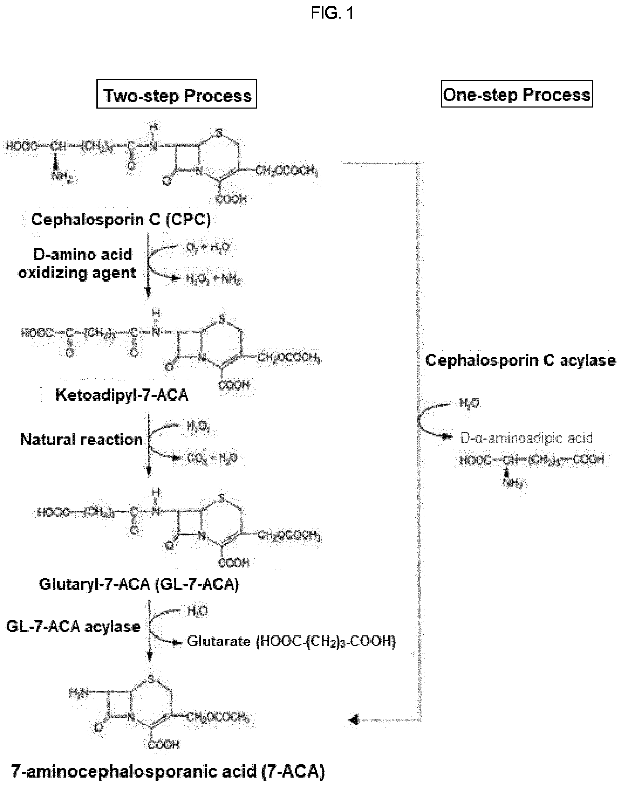

A typical enzymatic process consists of the following two steps: in the first step, CPC is converted into glutaryl-7-aminocephalosporanic acid (hereinafter referred to as "GI-7-ACA") through the enzymatic reaction of D-amino acid oxidase (hereinafter referred to as "DAO"); and the second step is adapted to convert GI-7-ACA into 7-ACA through the enzymatic reaction of GI-7-ACA acylase (see the left reaction scheme in FIG. 1).

However, the two-step enzymatic process suffers from the disadvantage of being low in production yield because the hydrogen peroxide produced in the enzymatic reaction of the first step attacks the substrate (CPC), the reaction product (GI-7-ACA), and DAO.

Suggested as an alternative for solving the problem is a one-step enzymatic process of producing 7-ACA directly from CPC without conducting the first step (see the right reaction scheme in FIG. 1). To this end, there is a need for CPC acylase that catalyzes the direct production of 7-ACA from CPC. Since CPC acylase does not exist in nature, it is synthesized by modifying various types of GI-7-ACA acylase (also known as glutaryl amidase (GA)) that naturally occur. However, GI-7-ACA acylase is poor in activity for CPC.

There is therefore a need for the development of CPC acylase that has an excellent enzymatic activity for CPC.

### SUMMARY OF THE INVENTION

Provided herein are a CPC acylase with improved enzymatic activity for cephalosporin C (hereinafter referred to as "CPC") and a use thereof.

An aspect provides a mutant CPC acylase, derived from a CPC acylase including an alpha-subunit of SEQ ID NO: 3 and a beta-subunit of SEQ ID NO: 4, wherein the mutant CPC acylase includes mutations by substitution of
(i) alanine at position 11 in the alpha-subunit of SEQ ID NO: 3 (A11α);
(ii) glycine at position 24 in the alpha-subunit of SEQ ID NO: 3 (G24α);
(iii) alanine at position 136 in the beta-subunit of SEQ ID NO: 4 (A136β); and
(iv) isoleucine at position 179 in the beta-subunit of SEQ ID NO: 4 (I179β); and optionally,
(v) histidine at position 453 in the beta-subunit of SEQ ID NO: 4 (H453β),
with an amino acid different from the corresponding original amino acid residue as further specified in the appended claims.

In one embodiment, the mutant CPC acylase including an alpha-subunit of SEQ ID NO: 3 and a beta-subunit of SEQ ID NO: 4 includes mutations by
(i-1) substitution of A11α in the alpha-subunit of SEQ ID NO: 3 with asparagine (N) (A11αN);
(ii-1) substitution of G24α in the alpha-subunit of SEQ ID NO: 3 with aspartic acid (D) (G24αD);
(iii-1) substitution of A136β in the beta-subunit of SEQ ID NO: 4 with threonine (T) (A136βT);
(iv-1) substitution of I179β in the beta-subunit of SEQ ID NO: 4 with tyrosine (Y) (I179βY); and
(v-1) substitution of H453β in the beta-subunit of SEQ ID NO: 4 with threonine (T) (H453βT).

In another embodiment, the mutant CPC acylase including an alpha-subunit of SEQ ID NO: 3 and a beta-subunit of SEQ ID NO: 4 may further include, in addition to the aforementioned amino acid substitutions, a mutation by at least one (one, two, three, four, or five, e.g., five) selected from the following substitutions in the beta-subunit of SEQ ID NO: 4:
(vi) substitution of isoleucine at position 45 (I45ß) with an amino acid different therefrom, for example, (vi-1) I45ßV;
(vii) substitution of phenylalanine at position 58 (F58ß) with an amino acid different therefrom, for example, (vii-1) F58ßV;
(viii) substitution of tyrosine at position 153 (Y153ß) with an amino acid different therefrom, for example, (viii-1) Y153ßT;
(ix) substitution of phenylalanine at position 177 (F177ß) with an amino acid different therefrom, for example, (ix-1) F177ßL; and
(x) substitution of valine at position 382 (V382ß) with an amino acid different therefrom, for example, (x-1) V382ßL.

Another aspect provides a nucleic acid sequence coding for the mutant CPC acylase described in the foregoing, a recombinant expression vector carrying the nucleic acid sequence, and a recombinant cell anchoring (comprising) the nucleic acid sequence and/or the recombinant expression vector thereat.

Another aspect provides a composition for production of 7-aminocephalosporanic acid (7-ACA) or a salt thereof, the composition including the mutant CPC acylase described above, a nucleic acid molecule coding for the mutant CPC acylase, a recombinant expression vector carrying the nucleic acid molecule, a recombinant cell comprising the nucleic acid molecule and/or the recombinant expression vector, and a culture of the recombinant cell.

Another aspect provides a use of at least one, selected from the group consisting of the mutant CPC acylase described above, a nucleic acid molecule coding for the mutant CPC acylase, a recombinant expression vector carrying the nucleic acid molecule, a recombinant cell comprising the nucleic acid molecule and/or the recombinant expression vector, and a culture of the recombinant cell, for producing 7-aminocephalosporanic acid (7-ACA) or a salt thereof.

Another aspect provides a method for producing 7-aminocephalosporanic acid (7-ACA) or a salt thereof, using at least one selected from the group consisting of the mutant CPC acylase described above, a nucleic acid molecule coding for the mutant CPC acylase, a recombinant expression vector carrying the nucleic acid molecule, a recombinant cell comprising the nucleic acid molecule and/or the recombinant expression vector, and a culture of the recombinant cell as further described in the claims.

### DETAILED DESCRIPTION

Provided herein are CPC acylase having an improved enzymatic activity for cephalosporin C (hereinafter referred to as "CPC") and a use thereof.

### Definition of Terms

As used herein, the phrase that a polynucleotide (interchangeably used with "gene" or "nucleic acid molecule") or a polypeptide (interchangeably used with "protein" or "enzyme") comprises (or includes) or a "specific nucleic acid sequence" or an "amino acid sequence" or consists or is composed (essentially) of a "specific nucleic acid sequence" or an "amino acid sequence" means that the polynucleotide or a polypeptide essentially contains the specific nucleic acid sequence or amino acid sequence therein, or is construed to include or comprise a "substantially identical sequence" resulting from the impartment of a mutation (deletion, substitution, alteration, and/or addition) to the specific nucleotide or amino acid sequence to the extent of retaining the original function and/or desired function of the polynucleotide or polypeptide.

In an embodiment, a polynucleotide or a polypeptide "includes (comprises) a specific nucleic acid sequence or an amino acid sequence" or "(essentially) consists of or represented by a specific nucleic acid sequence or an amino acid sequence" may mean that the polynucleotide or the polypeptide
(i) essentially contains the specific nucleic acid sequence or amino acid sequence, or
(ii) essentially consists of or contains a nucleic acid sequence or amino acid sequence having 60% or higher, 70% or higher, 80% or higher, 85% or higher, 90% or higher, 91 % or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity with the specific nucleic acid sequence or amino acid sequence and retains a desired function. Herein, the desired function may be a CPC acylase function (e.g., catalytic activity of hydrolyzing CPC and/or producing 7-ACA from CPC) (for the amino acid sequence), or a function of coding for a protein having such a CPC acylase function.

As used herein, the term "sequence identity" refers to a degree of match between two nucleic acid sequences or between two amino acid sequences and may be expressed as a percentage (%). As for identity with nucleic acid sequences, for example, its determination can be made using the algorithm BLAST (see: Karlin and Altschul, Pro. Natl. Acad. Sci. USA, 90, 5873, 1993) or the algorithm FASTA developed by Pearson (see: Methods Enzymol., 183, 63, 1990). Based on the algorithm BLAST, programs called BLASTN or BLASTX have been developed (see: http://www.ncbi.nlm.nih.gov).

As used herein, the term "amino acid residue at a specific position in the amino acid sequence of a protein (enzyme)" means an amino acid residue at a specific position in the amino acid sequence or an amino acid residue at a corresponding position in a homologous isotype protein and/or a heterologous protein having the same enzymatic activity as the protein.

When the first N-terminal amino acid residue in the amino acid sequence of a protein provided herein is methionine, it may be the residue contained in a natural state or may be synthesized in a recombinant process. Methionine, if absent at position 1 in the amino acid sequence of a protein, may be added as the first N-terminal residue during protein production in a recombinant manner.

As used herein, the term "about" is intended to encompass a numerical value that is identical to or numerical values that lie within the range of a certain variation of the following number, for example, within the range of a variation of ±0.5, ±0.4, ±0.3, 10.2, ±0.1, ±0.05, or ±0.01 of the given value, but with no limitations thereto.

Below, a detailed description will be given of the present disclosure.

### Mutant CPC Acylase

The present description provides a mutant CPC acylase with improved enzymatic activity. The mutant CPC acylase may result from the modification of imparting CPC acylase activity to conventional GI-7-ACA acylase.

GI-7-ACA acylase, used in the typical two-step enzymatic process (see the left reaction scheme in FIG. 1), is found in various soil microorganisms that are classified into five groups as in Table 1, below (classification of GI-7-ACA acylase) (Sonawane, Crit. Rev. Biotech. 26: 95-120, 2006):

**TABLE 1**

| Group | Representative Strain/Enzyme |
|---|---|
| Group I | *Pseudomonas* sp. A14 |
| Group II-A | *Bacillus lacteroporus* J 1 |
| Group II-B | *Pseudomonas* sp. GK16 |
| Group II-C | *Pseudomonas* sp. SE83 Acyl |
| Group III | *Pseudomonas* sp. N176 |

On the basis of the classification in Table 1, the proteins within the same group are very similar in size, amino acid sequence, substrate specificity, and reactivity whereas large different characteristics are found between proteins in different groups.

GI-7-ACA acylases in Group III are disadvantageous in that their activity is easily inhibited by 7-ACA and inorganic salts and the enzymatic reaction stability is poor. Even the modified enzymes derived from GI-7-ACA acylases in Group III retain the drawbacks. In contrast, since GI-7-ACA acylases in Group II-B are free of the drawbacks of enzymes in Group III, modification of the GI-7-ACA acylase in Group II-B allows for the development of a mutant enzyme applicable advantageously to the one-step enzymatic process for production of 7-ACA (see the right reaction scheme in FIG. 1).

Most GI-7-ACA acylases are composed of an alpha-subunit, a spacer peptide, and a beta-subunit in that order. The basic gene (ga gene) used in the present disclosure is transcribed and translated into a polypeptide composed of an inactive single chain with a size of about 77 kDa. Then, the spacer peptide is processed by cleavage to form a dimer consisting of an 18 kDa alpha-subunit (SEQ ID NO: 3) and a 58 kDa beta-subunit (SEQ ID NO: 4).

Accordingly, the mutant CPC acylase of the present description may result from a modification made to GI-7-ACA acylase in Group II-B. The GI-7-ACA acylase in Group II-B may be a GI-7-ACA acylase derived from *Pseudomonas* sp. strain, for example, *Pseudomonas* sp. Strain GK16.

The *Pseudomonas* sp. GK16-derived, wild-type GI-7-ACA acylase (SEQ ID NO: 1) may be, for example, encoded by the nucleic acid molecule of SEQ ID NO: 2, or may be in the form of a dimer including an 18 kDa alpha subunit (SEQ ID NO: 3) and a 58-kDa beta-subunit (SEQ ID NO: 4) which are derived from a 77 kDa inactive monomeric chain including an alpha-subunit (e.g., SEQ ID NO: 3), a spacer peptide (e.g., SEQ ID NO: 5), and beta-subunit (e.g., SEQ ID NO: 4) in that order from the N terminus which is processed into the dimeric chain by cleaving the spacer peptide. Point mutation may be introduced into one or more residues on the alpha-subunit and/or beta-subunit to provide a mutant having CPC acylase activity.

An aspect provides a mutant CPC acylase, derived from a CPC acylase including an alpha-subunit of SEQ ID NO: 3 and a beta-subunit of SEQ ID NO: 4, wherein the mutant CPC acylase includes mutations by substitution of:
(i) alanine at position 11 in the alpha-subunit of SEQ ID NO: 3 (A11α);
(ii) glycine at position 24 in the alpha-subunit of SEQ ID NO: 3 (G24α);
(iii) alanine at position 136 in the beta-subunit of SEQ ID NO: 4 (A136β); and
(iv) isoleucine at position 179 in the beta-subunit of SEQ ID NO: 4 (I179β); and optionally,
(v) histidine at position 453 in the beta-subunit of SEQ ID NO: 4 (H453β),
with an amino acid different from the corresponding original amino acid residue as further specified in the claims.

Another aspect provides a mutant CPC acylase, derived from a CPC acylase including an alpha-subunit of SEQ ID NO: 3 and a beta-subunit of SEQ ID NO: 4, wherein the mutant CPC acylase includes mutations by substitution
(i) alanine at position 11 in the alpha-subunit of SEQ ID NO: 3 (A11α);
(ii) glycine at position 24 in the alpha-subunit of SEQ ID NO: 3 (G24α);
(iii) alanine at position 136 in the beta-subunit of SEQ ID NO: 4 (A136β); and
(iv) isoleucine at position 179 in the beta-subunit of SEQ ID NO: 4 (I179β); and
with an amino acid different from the corresponding original amino acid residue as further specified in the claims.

The amino acid for substitution for amino acid residues on the subunits may be selected from the group consisting of alanine (A, Ala), asparagine (N, Asn), threonine (T, Thr), glutamic acid (E, Glu), serine (S, Ser), valine (V, Val), isoleucine (I, Ile), leucine (L, Leu), aspartic acid (D, Asp), cysteine (C, Cys), glutamine (Q, Gln), methionine (M, Met), phenylalanine (F, Phe), proline (P, Pro), tryptophane (W, Trp), tyrosine (Y, Tyr), arginine (R, Arg), histidine (H, His), lysine (K, Lys), and glycine (G, Gly) and is different from the corresponding original amino acid residues.

In one embodiment, the mutant CPC acylase including an alpha-subunit of SEQ ID NO: 3 and a beta-subunit of SEQ ID NO: 4 includes mutations by
(i-1) substitution of A11α in the alpha-subunit of SEQ ID NO: 3 with asparagine (N) (A11αN);
(ii-1) substitution of G24α in the alpha-subunit of SEQ ID NO: 3 with aspartic acid (D) (G24αD);
(iii-1) substitution of A136β in the beta-subunit of SEQ ID NO: 4 with threonine (T) (A136βT);
(iv-1) substitution of I179β in the beta-subunit of SEQ ID NO: 4 with tyrosine (Y) (I179βY); and
(v-1) substitution of H453β in the beta-subunit of SEQ ID NO: 4 with threonine (T) (H453βT).

In one embodiment, the mutant CPC acylase including an alpha-subunit of SEQ ID NO: 3 and a beta-subunit of SEQ ID NO: 4 includes mutations by
(i-1) substitution of A11α in the alpha-subunit of SEQ ID NO: 3 with asparagine (N) (A11αN);
(ii-1) substitution of G24α in the alpha-subunit of SEQ ID NO: 3 with aspartic acid (D) (G24αD);
(iii-1) substitution of A136β in the beta-subunit of SEQ ID NO: 4 with threonine (T) (A136βT); and
(iv-1) substitution of I179β in the beta-subunit of SEQ ID NO: 4 with tyrosine (Y) (I179βY).

In another embodiment, the mutant CPC acylase including an alpha-subunit of SEQ ID NO: 3 and a beta-subunit of SEQ ID NO: 4 may further include, in addition to the amino acid substitutions (i) to (iv) (e.g., amino acid substitutions (i-1), (ii-1), (iii-1), and (iv-1)), a mutation by substitution of (v) histidine at position 453 in the beta-subunit of SEQ ID NO: 4 (H453β) with an amino acid different therefrom, for example, a mutation by (v-1) substitution of H453β in the beta-subunit of SEQ ID NO: 4 with threonine (T) (H453βT).

In another embodiment, the mutant CPC acylase including an alpha-subunit of SEQ ID NO: 3 and a beta-subunit of SEQ ID NO: 4 may further include, in addition to the aforementioned amino acid substitutions (i) to (v) (e.g., amino acid substitutions (i-1), (ii-1), (iii-1), (iv-1), and (v-1)), a mutation by at least one (one, two, three, four, or five, e.g., five) selected from the group consisting of the following substitutions in the beta-subunit of SEQ ID NO: 4:
(vi) substitution of isoleucine at position 45 (I45ß) with an amino acid different therefrom, for example, (vi-1) I45ßV;
(vii) substitution of phenylalanine at position 58 (F58ß) with an amino acid different therefrom, for example, (vii-1) F58ßV;
(viii) substitution of tyrosine at position 153 (Y153ß) with an amino acid different therefrom, for example, (viii-1) Y153ßT;
(ix) substitution of phenylalanine at position 177 (F177ß) with an amino acid different therefrom, for example, (ix-1) F177ßL; and
(x) substitution of valine at position 382 (V382ß) with an amino acid different therefrom, for example, (x-1) V382ßL.

The mutant CPC acylase including an alpha-subunit of SEQ ID NO: 3 and a beta-subunit of SEQ ID NO: 4 and including a mutation by amino acid substitutions (vi) to (x) (e.g., amino acid substitutions (vi-1), (vii-1), (viii-1), (ix-1), and (x-1)) retains the CPC acylase activity, but exhibits more enhanced CPC acylase activity and/or heat stability when further mutated by the amino acid substitutions (i) to (v) (e.g., amino acid substitutions (i-1), (ii-1), (iii-1), (iv-1), and (v-1)).

In a first embodiment, the mutant CPC acylase including an alpha-subunit of SEQ ID NO: 3 and a beta-subunit of SEQ ID NO: 4 may-includes a mutation by the following substitutions:
(i) substitution of the amino acid residue alanine at position 11 in the alpha-subunit of SEQ ID NO: 3 (A11α) with a different amino acid, for example, (i-1) A11αN **-**
(ii) substitution of the amino acid residue glycine at position 24 in the alpha-subunit of SEQ ID NO: 3 (G24α) with a different amino acid, for example, (ii-1) G24αD -
(iii) substitution of the amino acid residue alanine at position 136 in the beta-subunit of SEQ ID NO: 4 (A136β) with a different amino acid, for example, (iii-1) A136βT, and
(iv) substitution of the amino acid residue isoleucine at position 179 in the beta-subunit of SEQ ID NO: 4 (I179β) with a different amino acid, for example, (iv-1) I179βY.

In a further embodiment, the mutant CPC acylase including an alpha-subunit of SEQ ID NO: 3 and a beta-subunit of SEQ ID NO: 4 may include a mutation by the following substitution:
(v) substitution of the amino acid residue histidine at position 453 in the beta-subunit of SEQ ID NO: 4 (H453β) with a different amino acid, for example, (v-1) H453βT.

In a further embodiment, the mutant CPC acylase including an alpha-subunit of SEQ ID NO: 3 and a beta-subunit of SEQ ID NO: 4 may-includes mutations by the following substitutions:
(i) substitution of the amino acid residue alanine at position 11 in the alpha-subunit of SEQ ID NO: 3 (A11α)) with a different amino acid, for example, (i-1) A11αN;
(ii) substitution of the amino acid residue glycine at position 24 in the alpha-subunit of SEQ ID NO: 3 (G24α)) with a different amino acid, for example, (ii-1) G24αD;
(iii) substitution of the amino acid residue alanine at position 136 in the beta-subunit of SEQ ID NO: 4 (A136β) with a different amino acid, for example, (iii-1) A136βT; and
(iv) substitution of the amino acid residue isoleucine at position 179 in the beta-subunit of SEQ ID NO: 4 (I179β) with a different amino acid, for example, (iv-1) I179βY.

In a further embodiment, the mutant CPC acylase including an alpha-subunit of SEQ ID NO: 3 and a beta-subunit of SEQ ID NO: 4 may-includes mutations by the following substitutions:
(i) substitution of the amino acid residue alanine at position 11 in the alpha-subunit of SEQ ID NO: 3 (A11α)) with a different amino acid, for example, (i-1) A11αN;
(ii) substitution of the amino acid residue glycine at position 24 in the alpha-subunit of SEQ ID NO: 3 (G24α)) with a different amino acid, for example, (ii-1) G24αD;
(iii) substitution of the amino acid residue alanine at position 136 in the beta-subunit of SEQ ID NO: 4 (A136β) with a different amino acid, for example, (iii-1) A136βT;
(iv) substitution of the amino acid residue isoleucine at position 179 in the beta-subunit of SEQ ID NO: 4 (I179β) with a different amino acid, for example, (iv-1) I179βY; and optionally
(v) substitution of the amino acid residue histidine at position 453 in the beta-subunit of SEQ ID NO: 4 (H453β) with a different amino acid, for example, (v-1) H453βT.

In another aspect, the mutant CPC acylase according to any of the above embodiments may include an additional mutation by at least one (one, two, three, four, or five, e.g., five) selected from the group consisting of the following substitutions in the beta-subunit of SEQ ID NO: 4:
(vi) substitution of isoleucine at position 45 (I45ß) with an amino acid different therefrom, for example, (vi-1) I45ßV;
(vii) substitution of phenylalanine at position 58 (F58ß) with an amino acid different therefrom, for example, (vii-1) F58ßV;
(viii) substitution of tyrosine at position 153 (Y153ß) with an amino acid different therefrom, for example, (viii-1) Y153ßT;
(ix) substitution of phenylalanine at position 177 (F177ß) with an amino acid different therefrom, for example, (ix-1) F177ßL; and
(x) substitution of valine at position 382 (V382ß) with an amino acid different therefrom, for example, (x-1) V382ßL.

Due to at least one (one, two, three, four, or five) substitution selected from the group consisting of the amino acid substitutions (i) to (v) (e.g., the amino acid substitutions (i-1), (ii-1), (iii-1), (iv-1), and (v-1)) described in the foregoing, the mutant CPC acylase provided in the present description may have improvement in enzymatic activity for CPC (acylase activity), thermal stability, or both of them, compared to CPC acylases which do not have any of the mutations (substitutions).

In greater detail, compared to the mutant CPC acylase including mutation by at least one the amino acid substitutions (vi) to (x) (e.g., amino acid substitutions (vi-1), (vii-1), (viii-1), (ix-1), and (x-1)), but neither of the amino acid substitutions (i) to (V) (e.g., amino acid substitutions (i-1), (ii-1), (iii-1), (iv-1), and (v-1)), the mutant CPC acylase provided herein may be improved:
in terms of enzymatic activity for CPC by about 2 times or higher, about 2.5 times or higher, about 3 times or higher, about 3.5 times or higher, about 4 times or higher, about 4.5 times or higher, about 5 times or higher, about 5.5 times or higher, about 6 times or higher, about 6.5 times or higher, about 7 times or higher, about 7.5 times or higher, about 8 times or higher, about 8.5 times or higher, about 9 times or higher, about 9.5 times or higher, about 10 times or higher, about 10.5 times or higher, about 11 times or higher, about 11.5 times or higher, about 12 times or higher, about 12.5 times or higher, about 13 times or higher, about 13.5 times or higher, about 14 times or higher, about 14.5 times or higher, or about 15 times or higher (no particular restrictions are imparted to the upper limit, for example, up to about 100 times, about 90 times, about 80 times, about 70 times, about 60 times, about 50 times, about 40 times, about 30 times, or about 25 times, but with no limitations thereto), and/or
in terms of thermal stability by 1.05 times or greater, about 1.08 times or greater, about 1.1 times or greater, about 1.3 times or greater, about 1.5 times or greater, about 1.8 times or greater, about 2 times or greater, or about 2.3 times or greater (no particular restrictions are imparted to the upper limit, for example, up to about 10 times, about 9 times, about 8 times, about 7 times, about 6 times, about 5 times, about 4 times, about 3.5 times, or about 3 times, but with no limitations thereto).

In this regard, the term "enzymatic activity for CPC" refers to CPC acylase activity, and the unit for CPC acylase, accounting for enzymatic activity, is defined as the amount of the enzyme that catalyzes the conversion of one micromole of the substrate CPC per minute into 7-ACA. For instance, the acylase activity for CPC may mean the productivity of 7-ACA from CPC when the enzyme is allowed to react with the substrate CPC at 10°C for 16-18 hours after being left at 25°C for 2 hours and then at 10°C for 1 hour in a cell lysis buffer (1.25 mg/mL lysozyme, 1.25 mM EDTA, and 0.375%(w/v) Triton X-100). The term "thermal stability" may mean the productivity of 7-ACA from CPC at a high temperature (about 40°C or higher, e.g., about 45°C, about 50°C, about 55°C, or about 60°C), for example, when the enzyme is allowed to react with the substrate CPC at 10°C for 16-18 hours after being left at 25°C for 2 hours and then at 55°C for 1.5 hours in a cell lysis buffer (1.25 mg/mL lysozyme, 1.25 mM EDTA, and 0.375%(w/v) Triton X-100) (see Example 4 and Table 3).

In an embodiment, the mutant CPC acylase provided herein may be a non-naturally occurring enzyme, for example, produced in a recombinant manner or through chemical synthesis, but with no limitations thereto.

### Nucleic Acid Molecule, Recombinant Expression Vector, and Recombinant Cell

Another aspect provides a nucleic acid molecule coding for the mutant CPC acylase described in the foregoing. The nucleic acid molecule may code for the full-length sequence of the mutant CPC acylase or may be a combination of nucleic acid molecules coding for the alpha-subunit and the beta-subunit in the mutant CPC, respectively.

Another aspect provides a recombinant vector carrying the nucleic acid molecule. The recombinant vector may be an expression vector that can express the nucleic acid molecule in a suitable host cell. The recombinant vector may be a single vector carrying a nucleic acid molecule coding for the full-length sequence of the mutant CPC acylase (for example, nucleic acid molecules coding for the alpha-subunit and the beta-subunit in one vector) or may be a combination of a first recombinant vector carrying a nucleic acid molecule coding for the alpha-subunit of the mutant CPC acylase and a second recombinant vector carrying a nucleic acid molecule coding for the beta-subunit of the mutant CPC acylase.

Another aspect provides a recombinant cell comprising the nucleic acid molecule and/or the recombinant vector. The recombinant cell may be obtained by introducing the nucleic acid molecule and/or recombinant vector into a suitable host cell.

In an embodiment, the recombinant cell may be an *Escherichia coli* strain, for example, *Escherichia coli* MC1061-pBC-TEP11 strain (accession number: KCTC 14821BP) resulting from introduction of the nucleic acid molecule into *Escherichia coli* MC1061, but with no limitations thereto.

The mutant CPC acylase may be produced by culturing the recombinant cell in a suitable medium under an appropriate condition.

In an embodiment, the mutant CPC acylase may be produced by culturing a host cell transformed with a recombinant vector carrying a nucleic acid molecule coding for the full-length sequence of the mutant CPC acylase in a suitable medium and condition. In another embodiment, the mutant CPC acylase may be produced by culturing a host cell transformed with a recombinant expression vector carrying a gene coding for the alpha-subunit of the mutant CPC acylase or a gene having a sequence functionally equivalent thereto and a host cell transformed with a recombinant expression vector carrying a gene coding for the beta-subunit of the mutant CPC acylase or a gene having a sequence functionally equivalent thereto in respective suitable media and conditions to afford corresponding CPC acylase subunit proteins, followed by combining the two subunit proteins in vitro.

The mutant CPC acylase prepared as in the foregoing according to the present disclosure may be used for producing the target product or may be purified before use. The isolation and purification of the mutant CPC acylase may be achieved using the already known properties of CPC acylase through various chromatographic protein separation methods as they are or in a manner modified for experimental purposes. Alternatively, the mutant CPC acylase can be purified by affinity chromatography using specific binding affinity, such as binding affinity between a histidine peptide and a nickel column component or between a cellulose binding domain (CBD) and a cellulose.

Also, the mutant CPC acylase of the present disclosure can be used in an immobilized state, but not in a free state. The mutant CPC acylase can be immobilized using a typical method known in the art. In this regard, a natural polymer such as cellulose, starch, dextran, agarose, etc.; a synthetic polymer such as polyacrylamide, polyacrylate, polymethacrylate, Eupergit C, etc.; or a mineral such as silica, bentonite, metal, etc. may be employed as a carrier. The mutant CPC acylase can be coupled to the carrier through a covalent bond, a hydrophobic bond, physical adsorption, microencapsulation, etc. In addition, the immobilization of the mutant CPC acylase in the carrier-enzyme conjugate form may be achieved through glutaraldehyde which forms covalent bonds between the cyanogen bromide of the carrier and the enzyme. Microbes containing the CPC acylase may be immobilized as they are, without separately purifying the enzyme. When such whole cell immobilization is employed, the cells may be punctured or a technique such as surface expression may be applied in order to increase the reactivity of the mutant CPC acylase contained therein.

In the coding regions of the nucleic acid sequences disclosed herein, various alterations or modifications may be made due to their codon degeneracy in consideration of the codons preferred by an organism in which the protein is to be expressed, provided that they do not change the amino acid sequence and/or function of the polypeptide expressed from the coding region.

The introduction of the nucleic acid molecule or vector may be carried out using a well-known transformation method appropriately selected by a person skilled in the art. As used herein, the term "transformation" means the introduction of a vector carrying a nucleic acid coding for a target protein (foreign protein) into a host cell in such a way that the protein encoded by the nucleic acid molecule is expressed in the host cell. So long as the transformed nucleic acid molecule is expressed in the host cell, it may be either integrated into the chromosome of the host cell and/or may exist extrachromosomally. Further, the nucleic acid molecule includes DNA and/or RNA encoding the target protein. The nucleic acid molecule may be introduced in any form as long as it can be introduced into the host cell and expressed therein. For example, the nucleic acid molecule may be introduced into the host cell via the form of an expression cassette that is a gene construct including all elements required for its autonomous expression. Typically, the expression cassette includes an expression regulatory element operatively linked to the nucleic acid molecule, such as a promoter, a transcriptional termination signal, a ribosome binding site, and/or a translation termination signal. The expression cassette may be in the form of a self-replicable expression vector. Also, the nucleic acid molecule as it is may be introduced into the host cell and operably linked to sequences required for expression in the host cell. As used herein, the term "operably linked" means a functional linkage between a nucleic acid molecule and an expression regulatory element (e.g., promoter) so as for the expression regulatory element to control (e.g., initiate) the transcription of the nucleic acid molecule encoding a target protein (foreign protein). The operative linkage may be carried out using a genetic recombination technique known in the art. By way of example, the linkage may be implemented by typical site-specific DNA cleavage and ligation, but is not limited thereto.

So long as it ensures the introduction of the nucleic acid molecule into a host cell (microorganism), any transformation may be used in the present disclosure. According to host cells, appropriate selection may be made of transformation techniques known in the art. Examples of the transformation methods known in the art include electroporation, calcium phosphate (CaPO₄) precipitation, calcium chloride (CaCl₂) precipitation, microinjection, polyethylene glycol (PEG)-mediated uptake, DEAE-dextran-mediated gene transfer, cationic liposome-mediated gene transfer, lipofection, lithium acetate-DMSO method, heat shock, particle gun bombardment, silicon carbide whiskers, and sonication, but are not limited thereto.

The introduction (insertion) of the nucleic acid molecule into the genome (chromosome) of a host cell may be conducted using a known method appropriately selected by a person skilled in the art. For instance, the nucleic acid molecule may be introduced using at least one selected from the group consisting of an RNA-guided endonuclease system or CRISPR system (e.g., (a) RNA-guided endonuclease (i.e., Cas9 protein, etc.), a gene coding therefor, or a vector carrying the gene; and (b) guide RNA (i.e., single guide RNA (sgRNA), etc.)), a DNA coding therefor or a mixture including a vector carrying the DNA (e.g., a mixture of RNA-guided endonuclease protein and guide RNA, etc.), a complex (e.g., ribonucleoprotein (RNP)), and a recombinant vector (e.g., vector carrying both an RNA-guided endonuclease-encoding gene and a guide RNA-encoding DNA), but with no limitations thereto.

As used herein, the term "vector" refers to a DNA construct including a nucleotide sequence of a nucleic acid molecule coding for a target protein, which is operatively linked to an appropriate expression regulatory sequence to express the target protein in a suitable host cell. The regulatory sequence may include a promoter that can initiate transcription, an optional operator sequence for regulating the transcription, a sequence encoding a suitable mRNA ribosome binding site, and/or a sequence regulating the termination of transcription and/or translation. After being transformed into the suitable host cell, the vector may replicate or function independently of the host genome or may be integrated into the genome of the host cell.

The vector available in the present invention is not particularly limited as long as it is able to replicate in the host cell, and any vector known in the art may be used. Examples of conventional vectors may include a natural or recombinant plasmid, cosmid, virus, and bacteriophage. For instance, pWE15, M13, MBL3, MBL4, IXII, ASHII, APII, t10, t11, Charon4A, or Charon21A may be used as a phage vector or cosmid vector, and examples of plasmid vectors include pBC types (e.g., pBC-KS(+)), pBR types (e.g., pBR322, pBR325), pUC types (e.g., pUC118 and pUC119), pBluescriptll types, pGEM types, pTZ types, pCL types, pET types (e.g., pET-22b(+)), Bacillus subtilis-derived plasmids (e.g., pUB110, pTP5), plasmids derived from animal viruses such as retrovirus, adenovirus, Vaccinia virus, etc., plasmids derived from insect viruses such as Baculoviruses, but with no limitations thereto.

Any type of single-cell organisms typically used may be available as the host cell. For example, the host cell may be selected from the group consisting of prokaryotic microbes such as various bacteria (e.g., Clostridia spp. Escherichia spp., etc.) and eukaryotic microbes such as yeasts. Selection may be made of, for example, Clostridia spp. microorganisms (e.g., Clostridium acetobutylicum, Clostridium beijerinckii, Clostridium saccharoperbutylacetonicum, and Clostridium saccharobutylicum) and Escherichia spp. microorganisms (e.g., Escherichia coli), but with no limitations thereto.

The vector available herein may be a well-known expression vector and/or a vector for inserting a nucleic acid molecule into the chromosome. The insertion of a nucleic acid molecule into the chromosome of a host cell may be implemented by any method known in the art, for example, homologous recombination or a CRISPR system, but with no limitations thereto. The vector may further include a selection marker for indicating insertion into the chromosome. The selection marker is adapted to select transformed cells, that is, to determine whether the polynucleotide is inserted or not, and may be selected from genes leading to selectable phenotypes such as drug resistance, auxotrophy, resistance to cytotoxic agents, or expression of surface proteins. In the presence of a selective agent, only the cells expressing the selection marker are alive or exhibit a different expression trait and thus can be selected.

### Production of 7-Aminocephalosporanic Acid (7-ACA)

Another aspect of the present description provides a composition for producing 7-aminocephalosporanic acid (7-ACA) or a salt thereof, the composition including at least one selected from the group consisting of the mutant CPC acylase described in the foregoing, a nucleic acid sequence coding for the mutant CPC acylase, a recombinant expression vector carrying the nucleic acid sequence, a recombinant cell comprising the nucleic acid sequence and/or the recombinant expression vector, and a culture of the recombinant cell.

Another aspect provides a use of at least one selected from the group consisting of the mutant CPC acylase described in the foregoing, a nucleic acid sequence coding for the mutant CPC acylase, a recombinant expression vector carrying the nucleic acid sequence, a recombinant cell comprising the nucleic acid sequence and/or the recombinant expression vector, and a culture of the recombinant cell for producing 7-ACA or a salt thereof.

Another aspect provides a method for producing 7-ACA or a salt thereof, using at least one selected from the group consisting of the mutant CPC acylase described in the foregoing, a nucleic acid sequence coding for the mutant CPC acylase, a recombinant expression vector carrying the nucleic acid sequence, a recombinant cell comprising the nucleic acid sequence and/or the recombinant expression vector, and a culture of the recombinant cell.

Another aspect provides a method for producing a compound of Chemical Formula 2 or a salt thereof, the method comprising a step of contacting at least one selected from the group consisting of the mutant CPC acylase described in the foregoing, a nucleic acid sequence coding for the mutant CPC acylase, a recombinant expression vector carrying the nucleic acid sequence, a recombinant cell comprising the nucleic acid sequence and/or the recombinant expression vector, and a culture of the recombinant cell with a compound of Chemical Formula 1 or a salt thereof: 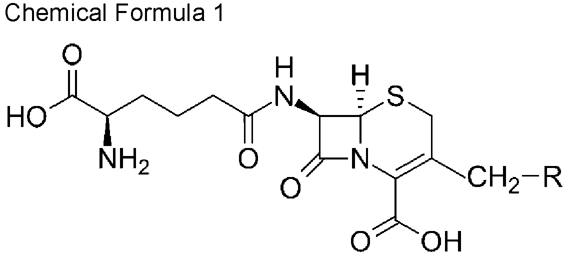 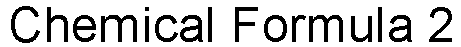 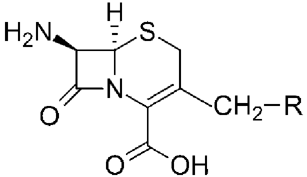 (wherein, R is acetoxy (-OCOCH₃), hydroxy (-OH), or hydrogen (-H) group).

The salt may be any type of pharmaceutically acceptable salts. For example, alkali metal salts (e.g., sodium salts, potassium salts, lithium salts, etc.) may be used, but with no limitations thereto. In an embodiment, the compound of Chemical Formula 1 may be a CPC substrate compound and the compound of Chemical Formula 2 may be 7-ACA.

The method, provided herein, for producing a compound of Chemical Formula 2 or a salt thereof, for example, 7-ACA or a salt thereof may be a one-step method for producing 7-ACA or a salt thereof directly from CPC.

That is, the method may not include a step of treating CPC with D-amino acid oxidase. Through the enzymatic reaction of D-amino acid oxidase, CPC is converted into glutaryl-7-aminocephalosporanic acid (hereinafter referred to as "GI-7-ACA") during which hydrogen peroxide is also generated and attacks the substrate (CPC), the reaction product (GI-7-ACA), and D-amino acid oxidase, thus lowering the production yield. In contrast, the method provided herein does not include the step of treating CPC with D-amino acid oxidase, but produces 7-ACA or a salt thereof directly from CPC, thereby solving the problems.

The recombinant cell is a strain for producing the mutant CPC acylase.

The compound of Chemical Formula 2 can be prepared by contacting the compound of Chemical Formula 1 with the mutant CPC acylase which may be within the mutant CPC acylase-producing strain or a culture of the mutant CPC acylase-producing strain (e.g., a liquid phase, semi-solid phase, or solid phase culture, or a culture of a concentrate or dried form) or in the form of a composition or may be in an isolated state or an immobilized state. The contact between the mutant CPC acylase and the compound of Chemical Formula 1 may be conducted in water or an aqueous solution (e.g., buffer). In this regard, conditions for the enzymatic reaction include a concentration of 1-500 mM for the compound of Chemical Formula 1, an amount of 0.1-100 U/mL for the mutant CPC acylase, a pH of 7-10 for the reaction mixture, a reaction time of 0.1-24 hours, and a reaction temperature of 4-40°C, but are not limited thereto. The compound of Chemical Formula 2 produced through the enzymatic reaction can be separated and/or purified by a typical method.

In addition, the compound of Chemical Formula 2 can be prepared in a cell through contact between the mutant CPC acylase and the compound of Chemical Formula 1. A recombinant expression vector carrying the mutant CPC acylase-encoding gene or a derivative having a functionally equivalent sequence is introduced into a strain able to biosynthesize the compound of Chemical Formula 1, such as E. coli or Acremonium chrysogenum, and the resulting transformant is cultured in a suitable medium under a proper condition, during which the biosynthesized compound of Chemical Formula 1 is spontaneously contacted with the mutant CPC acylase within the transformant to prepare the compound of Chemical Formula 2.

### EFFECT OF THE INVENTION

Provided herein is a mutant enzyme for producing 7-ACA, which is used as a raw material for cephalosporins. More specifically, a cephalosporin C acylase mutated to increase in enzymatic activity for cephalosporin C and/or thermal stability and a 7-ACA production method using same are provided, whereby the mutant CPC acylase of the present disclosure exhibits about 5-fold, for example, about 5- to about 21-fold higher enzymatic activity for CPC substrate and about 2.5-fold higher thermal activity than the conventional mutant CPC acylase (PM2). In addition, the technique of the present disclosure is a one-step process enjoying the advantage of effectively producing 7-ACA directly from CPC.

### BRIEF DESCRIPTION OF THE DRAWINGS

FIG. 1 is a schematic view showing a one-step and a two-step enzymatic process for producing 7-ACA from CPC.

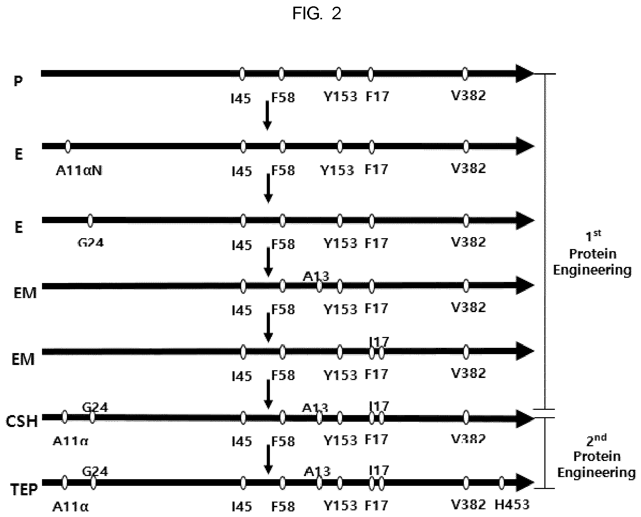

FIG. 2 is a schematic view showing a process of preparing a mutant CPC acylase according to an embodiment.

### EXAMPLES

A better understanding of the present disclosure may be obtained in light of the following examples which are set forth to illustrate the present disclosure.

### EXAMPLE 1: Mutant CPC Acylase Preparation and Activity Measurement

### 1-1. Construction of pBC-PM2 plasmid

To develop a mutant CPC acylase with improved enzymatic activity, a Pseudomonas sp. GK16 strain (Matsuda et. al., J. Bacteriol. 163: 1222-1228, 1985)-derived glutaryl amidase (GA) mutant (SEQ ID NO: 6)-encoding gene (hereinafter referred to as "pm gene"; SEQ ID NO: 7) was used as a base gene. The glutaryl amidase (GA) mutant (hereinafter referred to as "PM2 mutant") is a single chain polypeptide in which the wild-type alpha-subunit (SEQ ID NO: 3), the spacer (SEQ ID NO: 5), and a quintuple mutant (I45ßV/F58ßV/Y153ßT/F177ßL/V382ßL; SEQ ID NO: 8) of the beta-subunit (SEQ ID NO: 4) are linked through peptidyl bonds in that order from the N terminus (refer to Korean Patent No. 10-2014-0094150 A).

The pm gene was used as a precursor herein, and is expressed into a single chain polypeptide (SEQ ID NO: 6) in which the wild-type alpha-subunit of SEQ ID NO: 3 and the mutant beta-subunit of SEQ ID NO: 8 are linked to each other through the space of SEQ ID NO: 5 by peptidyl bonds, and then is developed into a mature active dimeric form consisting of the alpha-subunit and the beta-subunit through an autocatalytic process within the cell. The pm gene of SEQ ID NO: 7 was inserted into the pBC-KS (+) vector (Stratagene, USA) at the sites recognized by Xbal and Notl restriction enzymes recognition site to construct a pBC-PM2 plasmid for expressing the PM2 mutant. A concrete construction method is as follows.

The pm gene DNA product (about 2.1 kb in size) was digested with the restriction enzymes Xbal and Notl and purified using a purification kit (QIAquick Gel Extraction Kit; QIAGEN, Germany) to prepare a DNA to be inserted. Separately, a pBC KS(+) vector (Stratagene, USA) was also cleaved with Xbal and Notl and dephosphorylated with CIP (Calf Intestinal Alkaline Phosphatase) to give a vector DNA. The prepared DNA to be inserted was ligated to the vector DNA at 16°C for 12-16 hours in the presence of T4 DNA ligase (New England Biolabs, Sweden) and the ligation mixture was transformed into *E*. *coli* MC1061 by electroporation. The strain was spread on LB agar plates containing 20 µg/mL chloramphenicol antibiotic and statically incubated overnight at 30°C to select transformants. The plasmid was isolated from the selected transformant and subjected to base sequencing to identify the nucleotide sequence of the DNA insert. The resulting pBC-PM2 plasmid carrying the pm gene having the nucleotide sequence of SEQ ID NO: 7 was designed to express a PM2 mutant protein including the quintuple mutant (I45ßV/F58ßV/Y153ßT/F177ßL/V382ßL) of the beta-subunit.

### 1-2. Preparation of enzyme solution

The pBC-PM2 plasma-containing recombinant E. coli transformant prepared in Example 1-1 was assayed for productivity of CPC (cephalosporin C) acylase. For use in this assay, a CPC acylase coenzyme solution was prepared as follows: the E. coli transformant was inoculated into 3 mL of an LB broth (1% Bacto-tryptone, 0.5% yeast extract, 0.5% NaCl) containing 20 µg/mL chloramphenicol and cultured at 30°C for 16 hours while agitating at 200 rpm. Thereafter, 350 µL of the culture was inoculated into 35 mL of a fresh LB medium containing 20 µg/mL chloramphenicol and cultured at 25°C for 48 hours while agitating at 200 rpm. The resulting culture was centrifuged (4°C, 8000rpm, 10 min) to recover the cell mass which was then washed once with a 0.1M potassium phosphate buffer (pH 8.0). The cells were suspended in 35 mL of the same buffer, lysed at 4°C for 10 min by sonication (Vibra Cell VC750, Sonics & Materials Inc, USA), followed by centrifugation at 4°C and 13,500 rpm for 20 min. The supernatant thus formed was used as a mutant CPC acylase coenzyme solution. Below, the activity and thermal stability of CPC acylase were assayed using the enzyme solutions prepared from each strain according to the method described above.

### 1.3. Assay for CPC acylase activity

The activity of the CPC acylase prepared in Example 1.2 was measured using the Park et al. method (Park et al., Kor. J. Appl. Microbiol. Biotechnol. 23: 559-564, 1995), with a modification thereto, as follows.

The CPC substrate (purity 90%; Hayao, China) was dissolved at a concentration of 40 mM in a 0.1 M potassium phosphate buffer (pH 8.0) to give a substrate solution. To 20 µL of the CPC substrate solution was added 20 µL (microliter) of the enzyme solution prepared in Example 1.2. After the enzymatic reaction at 37°C for 5 min, the reaction was terminated by adding 200 µL of 50 mM NaOH-20%(w/v) glacial acetic acid (1:2, NaOH: glacial acetic acid, by volume). Then, centrifugation was conducted at 13000 rpm for 5 min and 200 µL of the supernatant thus formed was added with 40 µL of a solution of 0.5% (w/v) PDAB (p-dimethylaminobenzaldehyde; Sigma, USA) in methanol. Following the reaction for 10 min, absorbance was read at 415 nm. Quantitation was made by projecting the absorbance to a calibration curve for reference material. In this regard, one unit was defined as is defined as the amount of the enzyme that catalyzes the conversion of one micromole of the CPC substrate per minute into 7-aminocephalosporanic acid (7-ACA). Separately, the specific activity for CPC substrate was expressed as the activity unit of the enzyme per milligram of the total protein which was quantitated in the enzyme solution by the Bradford method (Bradford, M., Anal. Biochem. 72: 248-254, 1976).

As a result of the assay for CPC acylase activity, the recombinant *E*. *coli* MC1061(pBC-PM2) strain prepared in Example 1.1 was measured to express the CPC acylase at a productivity of about 1300 units/L.

### EXAMPLE 2: Preparation and Selection of CPC Acylase with High Activity

### 2.1. Construction of primary library by error-prone PCR

To prepare a mutant CPC acylase having improved CPC acylase activity, random mutations were artificially made to the nucleotide sequence of the pm gene described in Example 1.1. To this end, error-prone PCR was conducted to construct a library of mutants. Concrete processes of constructing a library of mutants are as follows.

Error-prone PCR was carried out using a Diversity PCR Random Mutagenesis kit (Clontech, USA) to cause 1-2 mutations per 1,000 bp. The PCR reaction mixture contained 1 ng/µL pBC-PM2 plasmid (Example 1.1) as a substrate DNA, 10 pmol of a T3 primer (SEQ ID NO: 15), 10 pmol of a T7 primer (SEQ ID NO: 16), 2 mM of dGTP, 50X Diversity dNTP mix, 10X TITANIUM Taq buffer, and TITANIUM Taq polymerase in a total volume of 50 µL. The PCR reaction condition was as follows: PCR was initiated by pre-denaturation at 95°C for 2 min and subjected to 18 cycles of denaturation at 95°C for 30 sec, annealing at 52°C for 30 sec, and extension at 68°C for 3 min, followed by post-extension at 68°C for 5 min. In this condition, amplification by the error-prone PCR resulted in a mutant DNA fragment with a size of about 2.1 kb.

The mutant genes obtained by error-prone PCR in the condition, that is, 2.1-kb PCR products were cut with the restriction enzymes Xbal and Notl and then purified using a purification kit (QIAquick Gel Extraction Kit; QIAGEN, Germany) to prepare a DNA to be inserted. Separately, a pBC-KS(+) vector (Stratagene, USA) was also cleaved with Xbal and Notl and dephosphorylated with CIP to give a vector DNA. The prepared DNA to be inserted was ligated to the vector DNA at 16°C for 12-16 hours in the presence of T4 DNA ligase (New England Biolabs, Sweden) and the ligation mixture was transformed into *E*. *coli* MC1061 by electroporation. The strain was spread on LB agar plates containing 20 µg/mL chloramphenicol antibiotic and statically incubated overnight at 30°C to construct a random mutant library.

From the mutant library, mutant CPC acylases with improved reactivity for CPC substrate were screened as follows.

*E. coli* MC1061 transformants including the mutant CPC acylase genes into which the additional mutations were introduced as described above were inoculated into 96-well plates, each well containing 160 µL of an LB broth supplemented with chloramphenicol, and was cultured at 30°C for 60-70 hours while agitation at 165 rpm. Thereafter, 20 µL of the culture taken from each well was transferred to new 96-well plates and added with 105 µL of a cell lysis buffer (1.25 mg/mL lysozyme, 1.25 mM EDTA, and 0.375%(w/v) Triton X-100), followed by incubation at 25°C for 2 hours and then at 10°C for one hour. To each well, 125 µL of a substrate solution of 50 mM CPC in a 25 mM potassium phosphate buffer (pH 8.0) was added to perform a hydrolysis reaction on the CPC substrate at 10°C for 16-18 hours. Following the hydrolysis reaction, 40 µL of the supernatant was transferred to each well of new 96-well plates. The enzymatic reaction was terminated with 100 µL of a reaction stop solution (acetic acid: 250mM NaOH, 2:1), added with 30 µL of a color development reagent (0.5% (w/v) PDAB in methanol), and left at room temperature for 10 min. Subsequently, absorbance was read on a 96-well plate reader at 415 nm, which is the wavelength indicative of the formation of 7-ACA, thereby screening mutants having improved activity for the CPC substrate.

As a result, selection was made of four mutants (EM5, EM9, EM17, and EM42) which had improved CPC acylase activity compared to PM2 (including I45ßV/F58ßVN153ßT/F177ßL/V382ßL mutations), from about 30,000 mutants. Base sequencing of the genes of the mutants revealed the substitution of the amino acid residue A11α (alanine at position 11 in the alpha-subunit) with asparagine (A11αN) in EM5 mutant enzyme, substitution of the amino acid residue G24α with aspartic acid (A11αD) in EM9 mutant enzyme, the substitution of the amino acid residue A136β (alanine at position 136 in the beta-subunit) with threonine (A136βT) in EM17 mutant enzyme, and the substitution of the amino acid residue I179β with tyrosine (I179βY) in EM42 mutant enzyme.

### 2.2. Construction of secondary mutant library by site-directed mutagenesis (DNA shuffling) - Selection of modified strain (CSH17)

To further increase CPC acylase activity, site-directed mutant libraries for the four amino acid residues (A11αN, G24αD, A136βT, and I179βY) were constructed on the basis of the activity of the modified strains obtained by the error-prone PCR in Example 2.1.

In brief, to construct mutant libraries for A11α and G24α, PCR was performed using M13-R primer (SEQ ID NO: 17) and SH24α-R primer (SEQ ID NO: 18) on the substrates PM2 and EM5 to obtain PCR products about 240 bp in size. Mutant libraries for G24α, A136β, and I179β were constructed by performing PCR using SH24α-F primer (SEQ ID NO: 19) and SH179β-R primer (SEQ ID NO: 20) on the substrates PM2 and EM17 to afford PCR products about 975 bp in size. For a mutant library for I179β, PCR was conducted using SH179β-F primer (SEQ ID NO: 21) and M13-F primer (SEQ ID NO: 22), with PM2 serving as a template, to obtain a PCR product about 1,120 bp in size.

In a final volume of 100 µL of the PCR reaction solution, a corresponding substrate DNA, primers, a pfu-x buffer, a dNTPs mix, and a pfu-x polymerase were contained. PCR was initiated by pre-denaturation at 95°C for 2 min and subjected to 18 cycles of denaturation at 95°C for 30 sec, annealing at 52°C for 30 sec, and extension at 68°C for 3 min, followed by post-extension at 68°C for 5 min.

The PCR products having sizes of about 240 bp, about 975 bp, and about 1,120 bp, thus obtained by PCR in the condition were mixed and subjected to PCR using T3 primer (SEQ ID NO: 15) and T7 primer (SEQ ID NO: 16) to amplify a 2.1-kb DNA fragment having multiple mutations. The 2.1-kb PCR product was inserted into a pBC-KS(+) vector DNA in the same manner as in Example 2.1 and then transformed into E. *coli* MC1061 to construct a site-directed mutant library.

In the site-directed mutant library, search was made for mutant CPC acylase having increased activity for CPC substrate in the same manner as in Example 2.1. As a result, a quadruple mutant (A11αN/G24αD/A136βT/1179βY) higher in CPC acylase activity than PM2 was selected in the same manner as in Example 2.1 and named CSH17. The CSH17 mutant enzyme (SEQ ID NO: 9) includes the alpha subunit of SEQ ID NO: 10 and the beta subunit of SEQ ID NO: 11.

### EXAMPLE 3: Preparation and Selection of Mutant Having Increased Thermal Stability Necessary for Enzymatic Stability - Construction of Tertiary Mutant Library by Error-Prone PCR - Selection of Modified Strain TEP11

To further increase the thermal stability of the modified strain CSH17 prepared in Example 2.2, a CSH17 (SEQ ID NO: 9)-encoding DNA was used as a template DNA for constructing an error-prone mutant library in the same manner as in Example 2.1.

In the mutant library, search was made for mutant CPC acylase with increased thermal stability in the same manner as in Example 2.1, except for the thermal treatment condition. In brief, error-prone PCR (see Example 2.1) was conducted on a CSH17(SEQ ID NO: 9)-encoding DNA as a template, to construct a random mutant library. The resulting *E*. *coli* MC1061 transformant which included a mutant CPC acylase gene having the mutation introduced thereinto was inoculated into 96-well plates, each well containing 160 µL of an LB broth supplemented with chloramphenicol and then cultured at 30°C for 60-70 hours while agitating at 165 rpm. Thereafter, 20 µL of the culture taken from each well was transferred to new 96-well plates and added with 105 µL of a cell lysis buffer (1.25 mg/mL lysozyme, 1.25 mM EDTA, and 0.375%(w/v) Triton X-100), followed by incubation at 25°C for 2 hours and then at 55°C for 1.5 hours for heat treatment. To each well, 125 µL of a substrate solution of 50 mM CPC in a 25 mM potassium phosphate buffer (pH 8.0) was added to perform a hydrolysis reaction on the CPC substrate at 10°C for 16-18 hours.

In the error-prone mutant library thus constructed, search was made for mutant CPC acylase having increased thermal stability. As a result, a strain having a mutation by substitution of H453β with threonine (H453βT) plus the CSH17 mutation (quadruple mutation A11αN/G24αD/A136βT/I179βY) was observed to exhibit high CPC acylase activity and remarkably increased thermal stability, compared to the CSH17 mutant, and named TEP11. TEP11 (SEQ ID NO: 12) includes the alpha subunit of SEQ ID NO:13 and the beta subunit of SEQ ID NO: 14.

Below, representative mutants of Examples 2 and 3 were assayed for CPC acylase activity and thermal stability.

### EXAMPLE 4: Assay for Activity and Thermal Stability of CPC Acylase Mutant

The CPC acylase mutants of Examples 2 and 3 were assayed for enzymatic activity and thermal stability in the same manners as in Examples 1.3 and 3, respectively. the results are summarized in Tables 2 and 3 (activity or thermal stability of mutant enzymes are expressed as relative values (folds) to that of PM2).

**TABLE 2**

| Mutated Residue in Mutant CPC Acylase and Comparison of Enzymatic Activity of | | |
|---|---|---|
| CPC Acylase | | |
| Mutant Enz. | Mutant Residue | Relative activity for CPC substrate (fold) |
| PM2 | I45ßV/F58ßV/Y153ßT/F177ßL/V382ßL | 1.0 |
| EM5 | **A11αN**/I45ßV/F58ßV/Y153ßT/F177ßL/V382ßL | 6.3 |
| EM9 | **G24αD**/I45ßV/F58ßV/Y153ßT/F177ßL/V382ßL | 4.5 |
| EM17 | I45RV/F58RV/**A136ß**TN153ßT/F177ßL/V382ß L | 5.2 |
| EM42 | I45ßV/F58ßV/Y153ßT/F177ßL/**I179ßY**/V382ßL | 6.9 |
| CSH17 | **A11αN/G24αD**/I45ßV/F58ßV/**A136ßT**/Y153ßT /F177ßL/**I179ßY**/V382βL | 15.7 |
| TEP11 | **A11αN/G24αD**/I45ßV/F58ßV/**A136ßT**/Y153ßT /F177ßL/**I179ßY**/V382ßL/**H453ßT** | 20.8 |

**TABLE 3**

| Mutated Residue in Mutant CPC Acylase and Comparison of Thermal Stability of CPC Acylase | | |
|---|---|---|
| Mutant Enz. | Mutant Residue | Relative activity for thermal stability (fold) |
| PM2 | I45ßV/F58ßV/Y153ßT/F177ßL/V382ßL | 1.0 |
| CSH17 | **A11αN/G24αD**/I45ßV/F58ßV/**A136ßT**/Y153ß T /F177ßL/**I179ßY**/V382ßL | 1.1 |
| TEP11 | **A11αN/G24αD**/I45ßV/F58ßV/**A136ßT**/Y153ß T /F177ßL/**I179ßY**/V382ßL/H453ßT | 2.4 |

Among the mutant enzymes, TEP11 mutant enzyme was observed to exhibit the highest activity and thermal stability. A pBC-TEP11 plasmid carrying a gene coding for the TEP11 mutant enzyme was transformed into an *E*. *coli* MC1061 strain which was then named *"Escherichia coli* MC1061-pBC-TEP11" and deposited with the Korean Collection for Type Cultures, located at Jeongeup-si. Jeollabuk-do, Korea, on December 13, 2021 (accession number: KCTC 14821BP).

### EXAMPLE 5: Comparison of Enzymatic Activity and Thermal Stability of CPC Acylase Mutants (vs. Wild-Type CPC acylase)

Mutant enzymes in which the five CPC acylate mutations (A11αN, G24αD, A136βT, I179βY, and H453βT) selected in Examples 2 and 3 were introduced into wild-type CPC acylase were assayed for enzymatic activity and thermal stability.

The mutants respectively including the five CPC acylase mutations (A11αN, G24αD, A136βT, I179βY, and H453βT) were prepared by site-directed mutagenesis.

Briefly, the five CPC acylase mutants (A11αN, G24αD, A136βT, I179βY, and H453βT) were prepared by PCR, with the wild-type CPC acylase (SEQ ID NO: 1) serving as a template. The primers in PCR are summarized in Table 4, below.

**TABLE 4**

| Mutant Enz. | Mutant residue | F Primer | R Primer |
|---|---|---|---|
| GA | WT | | |
| SM7 | I179ßY | GTTCCGACCTTTAACTA TGTTTATGCTGATCGTG( SEQ ID NO: 23) | CACGATCAGCATAAACA TAGTTAAAGGTCGGAAC (SEQ ID NO: 24) |
| SM10 | G24αD | GAAATCCTGTGGGATGA CTATGGTGTTCCGCATA TC(SEQ ID NO: 25) | GATATGCGGAACACCAT AGTCATCCCACAGGATT TC(SEQ ID NO: 26) |
| SM25 | A136ßT | CGTGCTGATGGTACCA CCGTTGCTGTTCGTGTT G(SEQ ID NO: 27) | CAACACGAACAGCAACG GTGGTACCATCAGCACG (SEQ ID NO: 28) |
| SM43 | A11αN | CCGCAGGCTCCGATCA | GCTACGCGGTTTATAAG |
| | | ATGCTTATAAACCGCGT AGC(SEQ ID NO: 29) | CATTGATCGGAGCCTGC GG(SEQ ID NO: 30) |
| SM52 | H453ßT | GTGCGTACCCCGGTTA CGGGTGAAACCTGGGT TGC(SEQ ID NO: 31) | GCAACCCAGGTTTCACC CGTAACCGGGGTACGC AC(SEQ ID NO: 32) |

In a final volume of 100 µL of the PCR reaction solution, a corresponding substrate DNA, primers, a pfu-x buffer, a dNTP mix, and a pfu-x polymerase were contained. PCR was initiated by pre-denaturation at 95°C for 2 min and subjected to 18 cycles of denaturation at 95°C for 30 sec, annealing at 52°C for 30 sec, and extension at 68°C for 3 min, followed by post-extension at 68°C for 5 min. The PCR product with a size of 2.1 kb, obtained in the condition, was inserted into a pBC-KS(+) vector DNA in the same manner as in Example 2.1 and transformed into E. coli MC1061 to give a site-directed mutant strain. For comparison, a recombinant strain was prepared by introducing wild-type CPC acylase (SEQ ID NO: 1) into *E*. *coli* MC1061.

The five CPC acylase mutant strains prepared above were assayed for enzymatic activity for CPC substrate and thermal stability with reference to the methods of Example 1.3 (enzymatic activity) and Example 3 (thermal stability, measured using the enzyme solution left at 40°C for 30 min), in comparison with the wild-type strain (SEQ ID NO: 1). The results are given in Table 5, below.

**TABLE 5**

| Mutant Enz. | Mutant Residue | Relative activity for CPC substrate (fold) | Relative activity for thermal stability (fold) |
|---|---|---|---|
| GA | Wild type | 1.00 | 1.00 |
| SM7 | I179ßY | 6.20 | 1.05 |
| SM10 | G24αD | 3.40 | 1.01 |
| SM25 | A136ßT | 2.85 | 0.98 |
| SM43 | A11αN | 4.70 | 1.07 |
| SM52 | H453ßT | 5.60 | 2.40 |

As can be seen in TABLE 5, the respective mutants having the five CPC acylase mutations (A11αN, G24αD, A136βT, I179βY, and H453βT) singly introduced thereinto exhibited about 2.9- to about 6.2-fold higher activity for CPC substrate and equivalent to about 2.4-fold higher thermal activity, compared to the wild-type (SEQ ID NO: 1).

### EXAMPLE 6: Comparison of Enzymatic Activity and Thermal Stability of CPC Acylase Mutant According to A11α Mutation

With reference to Example 5, CPC acylases were assayed for enzymatic activity and thermal stability according to the mutated residue for A11α.

In brief, site-directed mutagenesis was performed on the wild-type CPC acylase sequence (SEQ ID NO: 1) as a template to afford CPC acylase mutants A11αN, A11αP, A11αQ, A11αI, A11αV, A11αL, and A11αT in which the amino acid residue A11α was substituted with asparagine (N), proline (P), glutamine (Q), isoleucine (I), valine (V), leucine (L), and threonine (T), respectively.

The CPC acylase mutants prepared above and the wild-type CPC acylase were assayed for enzymatic activity for CPC substrate and thermal stability with reference to the methods of Examples 1.3 (enzymatic activity) and 3 (thermal stability, measured using the enzyme solution left at 40°C for 30 min).

The assay results for the CPC acylase mutants are expressed as relative values (folds) to those of the wild-type CPC acylase in Table 6, below.

**TABLE 6**

| Mutant Residue | Relative activity for CPC substrate (fold) | Relative activity for thermal stability (fold) |
|---|---|---|
| Wild type | 1.00 | 1.00 |
| A11αT | 0.95 | 0.88 |
| A11αN | 4.70 | 1.07 |
| A11αP | 2.20 | 1.00 |
| A11αQ | 2.90 | 1.00 |
| A11αI | 3.50 | 1.02 |
| A11αV | 3.80 | 1.03 |
| A11αL | 2.70 | 1.05 |

As shown in Table 6, the enzymatic activity for CPC substrate of the CPC acylase mutants A11αN, A11αP, A11αQ, A11αI, A11αV, and A11αL was superior to that of the wild type and about 2.3- to 5-fold higher than that of the CPC acylase mutant A11αT. In addition, the CPC acylase mutants A11αN, A11αP, A11αQ, A11αI, A11αV, and A11αL were equivalent to or higher than the wild type in terms of thermal stability whereas the CPC acylase mutant A11αT was lower in stability than the wild type. Furthermore, the CPC acylase mutants A11αN, A11αP, A11αQ, A11αI, A11αV, and A11αL exhibited about 1.14- to 1.22-fold improved stability, compared to the CPC acylase mutant A11αT.

From the data, it was understood that the mutant CPC acylase in which A11α was substituted with asparagine (N), proline (P), glutamine (Q), isoleucine (I), valine (V), or leucine (L) retained remarkably improved characteristics, compared to the wild type and the mutant in which the same amino acid residue (A11α) was substituted with a different amino acid (e.g., threonine (T)).

### [Accession Number]

Depositary Authority: Korean Collection for Type Culture
Accession Number: KCTC14821BP
Deposition date: December 13, 2021

The following 32 sequences are also encompassed by the application. These 32 sequences are submitted in parallel in the format required under WIPO ST. 26.
SEQ ID NO: 1
   length: 692
   type: PRT
   source: Artificial Sequence
   note: Protein (692 aa) of wild-type Gl-7-ACA acylase from Pseudomonas sp. GK16, comprising alpha- and beta-subunits and a spacer therebetween 
Met Glu Pro Thr Ser Thr Pro Gln Ala Pro Ile Ala Ala Tyr Lys Pro
Arg Ser Asn Glu Ile Leu Trp Asp Gly Tyr Gly Val Pro His Ile Tyr
Gly Val Asp Ala Pro Ser Ala Phe Tyr Gly Tyr Gly Trp Ala Gln Ala
Arg Ser His Gly Asp Asn Ile Leu Arg Leu Tyr Gly Glu Ala Arg Gly
Lys Gly Ala Glu Tyr Trp Gly Pro Asp Tyr Glu Gln Thr Thr Val Trp
Leu Leu Thr Asn Gly Val Pro Glu Arg Ala Gln Gln Trp Tyr Ala Gln
Gln Ser Pro Asp Phe Arg Ala Asn Leu Asp Ala Phe Ala Ala Gly Ile
Asn Ala Tyr Ala Gln Gln Asn Pro Asp Asp Ile Ser Pro Glu Val Arg
Gln Val Leu Pro Val Ser Gly Ala Asp Val Val Ala His Ala His Arg
Leu Met Asn Phe Leu Tyr Val Ala Ser Pro Gly Arg Thr Leu Gly Glu
Gly Asp Pro Pro Asp Leu Ala Asp Gln Gly Ser Asn Ser Trp Ala Val
Ala Pro Gly Lys Thr Ala Asn Gly Asn Ala Leu Leu Leu Gln Asn Pro
His Leu Ser Trp Thr Thr Asp Tyr Phe Thr Tyr Tyr Glu Ala His Leu
Val Thr Pro Asp Phe Glu Ile Tyr Gly Ala Thr Gln Ile Gly Leu Pro
Val Ile Arg Phe Ala Phe Asn Gln Arg Met Gly Ile Thr Asn Thr Val
Asn Gly Met Val Gly Ala Thr Asn Tyr Arg Leu Thr Leu Gln Asp Gly
Gly Tyr Leu Tyr Asp Gly Gln Val Arg Pro Phe Glu Arg Arg Gln Ala
Ser Tyr Arg Leu Arg Gln Ala Asp Gly Thr Thr Val Asp Lys Pro Leu
Glu Ile Arg Ser Ser Val His Gly Pro Val Phe Glu Arg Ala Asp Gly
Thr Ala Val Ala Val Arg Val Ala Gly Leu Asp Arg Pro Gly Met Leu
Glu Gln Tyr Phe Asp Met Ile Thr Ala Asp Ser Phe Asp Asp Tyr Glu
Ala Ala Leu Ala Arg Met Gln Val Pro Thr Phe Asn Ile Val Tyr Ala
Asp Arg Glu Gly Thr Ile Asn Tyr Ser Phe Asn Gly Val Ala Pro Lys
Arg Ala Glu Gly Asp Ile Ala Phe Trp Gln Gly Leu Val Pro Gly Asp
Ser Ser Arg Tyr Leu Trp Thr Glu Thr His Pro Leu Asp Asp Leu Pro
Arg Val Thr Asn Pro Pro Gly Gly Phe Val Gln Asn Ser Asn Asp Pro
Pro Trp Thr Pro Thr Trp Pro Val Thr Tyr Thr Pro Lys Asp Phe Pro
Ser Tyr Leu Ala Pro Gln Thr Pro His Ser Leu Arg Ala Gln Gln Ser
Val Arg Leu Met Ser Glu Asn Asp Asp Leu Thr Leu Glu Arg Phe Met
Ala Leu Gln Leu Ser His Arg Ala Val Met Ala Asp Arg Thr Leu Pro
Asp Leu Ile Pro Ala Ala Leu Ile Asp Pro Asp Pro Glu Val Gln Ala
Ala Ala Arg Leu Leu Ala Ala Trp Asp Arg Glu Phe Thr Ser Asp Ser
Arg Ala Ala Leu Leu Phe Glu Glu Trp Ala Arg Leu Phe Ala Gly Gln
Asn Phe Ala Gly Gln Ala Gly Phe Ala Thr Pro Trp Ser Leu Asp Lys
Pro Val Ser Thr Pro Tyr Gly Val Arg Asp Pro Lys Ala Ala Val Asp
Gln Leu Arg Thr Ala Ile Ala Asn Thr Lys Arg Lys Tyr Gly Ala Ile
Asp Arg Pro Phe Gly Asp Ala Ser Arg Met Ile Leu Asn Asp Val Asn
Val Pro Gly Ala Ala Gly Tyr Gly Asn Leu Gly Ser Phe Arg Val Phe
Thr Trp Ser Asp Pro Asp Glu Asn Gly Val Arg Thr Pro Val His Gly
Glu Thr Trp Val Ala Met Ile Glu Phe Ser Thr Pro Val Arg Ala Tyr
Gly Leu Met Ser Tyr Gly Asn Ser Arg Gln Pro Gly Thr Thr His Tyr
 
Ser Asp Gln Ile Glu Arg Val Ser Arg Ala Asp Phe Arg Glu Leu Leu
Leu Arg Arg Glu Gln Val Glu Ala Ala Val Gln Glu Arg Thr Pro Phe
Asn Phe Lys Pro
SEQ ID NO: 2
   length: 2079
   type: PRT
   source: Artificial Sequence
   note: Gene (2079 bp) encoding the proten of SEQ ID NO: 1
SEQ ID NO: 2 
Ala Thr Gly Gly Ala Ala Cys Cys Gly Ala Cys Cys Ala Gly Cys Ala
Cys Cys Cys Cys Gly Cys Ala Gly Gly Cys Thr Cys Cys Gly Ala Thr
Cys Gly Cys Thr Gly Cys Thr Thr Ala Thr Ala Ala Ala Cys Cys Gly
Cys Gly Thr Ala Gly Cys Ala Ala Cys Gly Ala Ala Ala Thr Cys Cys
Thr Gly Thr Gly Gly Gly Ala Thr Gly Gly Thr Thr Ala Thr Gly Gly
Thr Gly Thr Thr Cys Cys Gly Cys Ala Thr Ala Thr Cys Thr Ala Thr
Gly Gly Thr Gly Thr Thr Gly Ala Cys Gly Cys Thr Cys Cys Gly Ala
Gly Cys Gly Cys Thr Thr Thr Thr Thr Ala Thr Gly Gly Thr Thr Ala
Thr Gly Gly Cys Thr Gly Gly Gly Cys Thr Cys Ala Gly Gly Cys Thr
Cys Gly Thr Ala Gly Cys Cys Ala Thr Gly Gly Thr Gly Ala Thr Ala
Ala Cys Ala Thr Cys Cys Thr Gly Cys Gly Thr Cys Thr Gly Thr Ala
Thr Gly Gly Thr Gly Ala Ala Gly Cys Thr Cys Gly Thr Gly Gly Thr
Ala Ala Ala Gly Gly Cys Gly Cys Thr Gly Ala Ala Thr Ala Thr Thr
Gly Gly Gly Gly Thr Cys Cys Gly Gly Ala Thr Thr Ala Cys Gly Ala
Ala Cys Ala Gly Ala Cys Gly Ala Cys Cys Gly Thr Thr Thr Gly Gly
Cys Thr Gly Cys Thr Gly Ala Cys Cys Ala Ala Cys Gly Gly Thr Gly
Thr Thr Cys Cys Gly Gly Ala Ala Cys Gly Thr Gly Cys Thr Cys Ala
Gly Cys Ala Gly Thr Gly Gly Thr Ala Thr Gly Cys Thr Cys Ala Gly
Cys Ala Gly Ala Gly Cys Cys Cys Gly Gly Ala Thr Thr Thr Thr Cys
Gly Thr Gly Cys Thr Ala Ala Cys Cys Thr Gly Gly Ala Thr Gly Cys
Thr Thr Thr Thr Gly Cys Thr Gly Cys Thr Gly Gly Thr Ala Thr Cys
Ala Ala Cys Gly Cys Thr Thr Ala Thr Gly Cys Thr Cys Ala Gly Cys
Ala Gly Ala Ala Cys Cys Cys Gly Gly Ala Thr Gly Ala Thr Ala Thr
Cys Ala Gly Cys Cys Cys Gly Gly Ala Ala Gly Thr Thr Cys Gly Thr
Cys Ala Gly Gly Thr Thr Cys Thr Gly Cys Cys Gly Gly Thr Thr Ala
Gly Cys Gly Gly Thr Gly Cys Thr Gly Ala Thr Gly Thr Thr Gly Thr
Thr Gly Cys Thr Cys Ala Thr Gly Cys Thr Cys Ala Thr Cys Gly Thr
Cys Thr Gly Ala Thr Gly Ala Ala Cys Thr Thr Thr Cys Thr Gly Thr
Ala Thr Gly Thr Thr Gly Cys Thr Ala Gly Cys Cys Cys Gly Gly Gly
Thr Cys Gly Thr Ala Cys Cys Cys Thr Gly Gly Gly Thr Gly Ala Ala
Gly Gly Thr Gly Ala Thr Cys Cys Gly Cys Cys Gly Gly Ala Thr Cys
Thr Gly Gly Cys Thr Gly Ala Thr Cys Ala Gly Gly Gly Thr Ala Gly
Cys Ala Ala Cys Ala Gly Cys Thr Gly Gly Gly Cys Thr Gly Thr Thr
Gly Cys Thr Cys Cys Gly Gly Gly Thr Ala Ala Ala Ala Cys Cys Gly
Cys Thr Ala Ala Cys Gly Gly Thr Ala Ala Cys Gly Cys Thr Cys Thr
Gly Cys Thr Gly Cys Thr Gly Cys Ala Gly Ala Ala Cys Cys Cys Thr
Cys Ala Thr Cys Thr Gly Ala Gly Cys Thr Gly Gly Ala Cys Cys Ala
Cys Cys Gly Ala Thr Thr Ala Thr Thr Thr Ala Cys Cys Thr Ala
Thr Thr Ala Thr Gly Ala Ala Gly Cys Thr Cys Ala Thr Cys Thr Gly
Gly Thr Thr Ala Cys Cys Cys Cys Gly Gly Ala Thr Thr Thr Thr Gly
Ala Ala Ala Thr Cys Thr Ala Thr Gly Gly Thr Gly Cys Thr Ala Cys
 
Cys Cys Ala Gly Ala Thr Cys Gly Gly Thr Cys Thr Gly Cys Cys Gly
Gly Thr Thr Ala Thr Cys Cys Gly Thr Thr Thr Thr Gly Cys Thr Thr
Thr Thr Ala Ala Cys Cys Ala Gly Cys Gly Thr Ala Thr Gly Gly Gly
Thr Ala Thr Cys Ala Cys Cys Ala Ala Cys Ala Cys Cys Gly Thr Thr
Ala Ala Cys Gly Gly Thr Ala Thr Gly Gly Thr Thr Gly Gly Thr Gly
Cys Thr Ala Cys Cys Ala Ala Cys Thr Ala Thr Cys Gly Thr Cys Thr
Gly Ala Cys Cys Cys Thr Gly Cys Ala Gly Gly Ala Thr Gly Gly Thr
Gly Gly Thr Thr Ala Thr Cys Thr Gly Thr Ala Thr Gly Ala Thr Gly
Gly Thr Cys Ala Gly Gly Thr Thr Cys Gly Thr Cys Cys Gly Thr Thr
Thr Gly Ala Ala Cys Gly Thr Cys Gly Thr Cys Ala Gly Gly Cys Thr
Ala Gly Cys Thr Ala Thr Cys Gly Thr Cys Thr Gly Cys Gly Thr Cys
Ala Gly Gly Cys Thr Gly Ala Thr Gly Gly Thr Ala Cys Cys Ala Cys
Cys Gly Thr Thr Gly Ala Thr Ala Ala Ala Cys Cys Gly Cys Thr Gly
Gly Ala Ala Ala Thr Cys Cys Gly Thr Thr Cys Cys Ala Gly Cys Gly
Thr Thr Cys Ala Thr Gly Gly Thr Cys Cys Gly Gly Thr Thr Thr Thr
Thr Gly Ala Ala Cys Gly Thr Gly Cys Thr Gly Ala Thr Gly Gly Thr
Ala Cys Cys Gly Cys Thr Gly Thr Thr Gly Cys Thr Gly Thr Thr Cys
Gly Thr Gly Thr Thr Gly Cys Thr Gly Gly Thr Cys Thr Gly Gly Ala
Thr Cys Gly Thr Cys Cys Gly Gly Gly Thr Ala Thr Gly Cys Thr Gly
Gly Ala Ala Cys Ala Gly Thr Ala Thr Thr Thr Thr Gly Ala Thr Ala
Thr Gly Ala Thr Cys Ala Cys Cys Gly Cys Thr Gly Ala Thr Ala Gly
Cys Thr Thr Thr Gly Ala Thr Gly Ala Thr Thr Ala Thr Gly Ala Ala
Gly Cys Thr Gly Cys Thr Cys Thr Gly Gly Cys Thr Cys Gly Thr Ala
Thr Gly Cys Ala Gly Gly Thr Thr Cys Cys Gly Ala Cys Cys Thr Thr
Thr Ala Ala Cys Ala Thr Cys Gly Thr Thr Thr Ala Thr Gly Cys Thr
Gly Ala Thr Cys Gly Thr Gly Ala Ala Gly Gly Thr Ala Cys Cys Ala
Thr Cys Ala Ala Cys Thr Ala Thr Ala Gly Cys Thr Thr Thr Ala Ala
Cys Gly Gly Thr Gly Thr Thr Gly Cys Thr Cys Cys Gly Ala Ala Ala
Cys Gly Thr Gly Cys Thr Gly Ala Ala Gly Gly Thr Gly Ala Cys Ala
Thr Cys Gly Cys Thr Thr Thr Thr Thr Gly Gly Cys Ala Gly Gly Gly
Thr Cys Thr Gly Gly Thr Thr Cys Cys Gly Gly Gly Thr Gly Ala Thr
Ala Gly Cys Ala Gly Cys Cys Gly Thr Thr Ala Thr Cys Thr Gly Thr
Gly Gly Ala Cys Cys Gly Ala Ala Ala Cys Cys Cys Ala Thr Cys Cys
Gly Cys Thr Gly Gly Ala Thr Gly Ala Thr Cys Thr Gly Cys Cys Gly
Cys Gly Thr Gly Thr Thr Ala Cys Cys Ala Ala Cys Cys Cys Gly Cys
Cys Gly Gly Gly Thr Gly Gly Thr Thr Thr Thr Gly Thr Thr Cys Ala
Gly Ala Ala Cys Ala Gly Cys Ala Ala Cys Gly Ala Thr Cys Cys Gly
Cys Cys Gly Thr Gly Gly Ala Cys Cys Cys Cys Gly Ala Cys Cys Thr
Gly Gly Cys Cys Gly Gly Thr Thr Ala Cys Cys Thr Ala Thr Ala Cys
Cys Cys Cys Gly Ala Ala Ala Gly Ala Thr Thr Thr Thr Cys Cys Gly
Ala Gly Cys Thr Ala Thr Cys Thr Gly Gly Cys Thr Cys Cys Gly Cys
Ala Gly Ala Cys Cys Cys Cys Gly Cys Ala Thr Ala Gly Cys Cys Thr
Gly Cys Gly Thr Gly Cys Thr Cys Ala Gly Cys Ala Gly Ala Gly Cys
Gly Thr Thr Cys Gly Thr Cys Thr Gly Ala Thr Gly Ala Gly Cys Gly
Ala Ala Ala Ala Cys Gly Ala Thr Gly Ala Thr Cys Thr Gly Ala Cys
Cys Cys Thr Gly Gly Ala Ala Cys Gly Thr Thr Thr Thr Ala Thr Gly
Gly Cys Thr Cys Thr Gly Cys Ala Gly Cys Thr Gly Ala Gly Cys Cys
Ala Thr Cys Gly Thr Gly Cys Thr Gly Thr Thr Ala Thr Gly Gly Cys
Thr Gly Ala Thr Cys Gly Thr Ala Cys Cys Cys Thr Gly Cys Cys Gly
Gly Ala Thr Cys Thr Gly Ala Thr Cys Cys Cys Gly Gly Cys Thr Gly
Cys Thr Cys Thr Gly Ala Thr Cys Gly Ala Thr Cys Cys Gly Gly Ala
 
Thr Cys Cys Gly Gly Ala Ala Gly Thr Thr Cys Ala Gly Gly Cys Thr
Gly Cys Thr Gly Cys Thr Cys Gly Thr Cys Thr Gly Cys Thr Gly Gly
Cys Thr Gly Cys Gly Thr Gly Gly Gly Ala Thr Cys Gly Thr Gly Ala
Ala Thr Thr Thr Ala Cys Cys Ala Gly Cys Gly Ala Thr Ala Gly Cys
Cys Gly Thr Gly Cys Thr Gly Cys Thr Cys Thr Gly Cys Thr Gly Thr
Thr Thr Gly Ala Ala Gly Ala Ala Thr Gly Gly Gly Cys Thr Cys Gly
Thr Cys Thr Gly Thr Thr Thr Gly Cys Thr Gly Gly Thr Cys Ala Gly
Ala Ala Cys Thr Thr Thr Gly Cys Thr Gly Gly Thr Cys Ala Gly Gly
Cys Thr Gly Gly Cys Thr Thr Thr Gly Cys Thr Ala Cys Cys Cys Cys
Gly Thr Gly Gly Ala Gly Cys Cys Thr Gly Gly Ala Thr Ala Ala Ala
Cys Cys Gly Gly Thr Thr Ala Gly Cys Ala Cys Cys Cys Cys Gly Thr
Ala Thr Gly Gly Thr Gly Thr Thr Cys Gly Thr Gly Ala Thr Cys Cys
Gly Ala Ala Ala Gly Cys Thr Gly Cys Thr Gly Thr Thr Gly Ala Thr
Cys Ala Gly Cys Thr Gly Cys Gly Thr Ala Cys Cys Gly Cys Thr Ala
Thr Cys Gly Cys Thr Ala Ala Cys Ala Cys Cys Ala Ala Ala Cys Gly
Thr Ala Ala Ala Thr Ala Thr Gly Gly Thr Gly Cys Thr Ala Thr Cys
Gly Ala Thr Cys Gly Thr Cys Cys Gly Thr Thr Thr Gly Gly Thr Gly
Ala Thr Gly Cys Thr Ala Gly Cys Cys Gly Thr Ala Thr Gly Ala Thr
Cys Cys Thr Gly Ala Ala Cys Gly Ala Thr Gly Thr Thr Ala Ala Cys
Gly Thr Thr Cys Cys Gly Gly Gly Thr Gly Cys Thr Gly Cys Thr Gly
Gly Thr Thr Ala Thr Gly Gly Thr Ala Ala Cys Cys Thr Gly Gly Gly
Thr Ala Gly Cys Thr Thr Thr Cys Gly Thr Gly Thr Thr Thr Thr Thr
Ala Cys Cys Thr Gly Gly Ala Gly Cys Gly Ala Thr Cys Cys Gly Gly
Ala Thr Gly Ala Ala Ala Ala Cys Gly Gly Thr Gly Thr Gly Cys Gly
Thr Ala Cys Cys Cys Cys Gly Gly Thr Thr Cys Ala Thr Gly Gly Thr
Gly Ala Ala Ala Cys Cys Thr Gly Gly Gly Thr Thr Gly Cys Thr Ala
Thr Gly Ala Thr Cys Gly Ala Ala Thr Thr Thr Ala Gly Cys Ala Cys
Cys Cys Cys Gly Gly Thr Thr Cys Gly Thr Gly Cys Thr Thr Ala Thr
Gly Gly Thr Cys Thr Gly Ala Thr Gly Ala Gly Cys Thr Ala Thr Gly
Gly Thr Ala Ala Cys Ala Gly Cys Cys Gly Thr Cys Ala Gly Cys Cys
Gly Gly Gly Thr Ala Cys Cys Ala Cys Cys Cys Ala Thr Thr Ala Thr
Ala Gly Cys Gly Ala Thr Cys Ala Gly Ala Thr Cys Gly Ala Ala Cys
Gly Thr Gly Thr Thr Ala Gly Cys Cys Gly Thr Gly Cys Thr Gly Ala
Thr Thr Thr Thr Cys Gly Thr Gly Ala Ala Cys Thr Gly Cys Thr Gly
Cys Thr Gly Cys Gly Thr Cys Gly Thr Gly Ala Ala Cys Ala Gly Gly
Thr Thr Gly Ala Ala Gly Cys Thr Gly Cys Thr Gly Thr Thr Cys Ala
Gly Gly Ala Ala Cys Gly Thr Ala Cys Cys Cys Cys Gly Thr Thr Thr
Ala Ala Cys Thr Thr Thr Ala Ala Ala Cys Cys Gly Thr Gly Ala
SEQ ID NO: 3
   length: 158
   type: PRT
   source: Artificial Sequence
   note: Alpha-subunit (158 aa) of wild-type Gl-7-ACA acylase protein from Pseudomonas sp. GK16
SEQ ID NO: 3 
Glu Pro Thr Ser Thr Pro Gln Ala Pro Ile Ala Ala Tyr Lys Pro Arg
Ser Asn Glu Ile Leu Trp Asp Gly Tyr Gly Val Pro His Ile Tyr Gly
Val Asp Ala Pro Ser Ala Phe Tyr Gly Tyr Gly Trp Ala Gln Ala Arg
Ser His Gly Asp Asn Ile Leu Arg Leu Tyr Gly Glu Ala Arg Gly Lys
Gly Ala Glu Tyr Trp Gly Pro Asp Tyr Glu Gln Thr Thr Val Trp Leu
 
Leu Thr Asn Gly Val Pro Glu Arg Ala Gln Gln Trp Tyr Ala Gln Gln
Ser Pro Asp Phe Arg Ala Asn Leu Asp Ala Phe Ala Ala Gly Ile Asn
Ala Tyr Ala Gln Gln Asn Pro Asp Asp Ile Ser Pro Glu Val Arg Gln
Val Leu Pro Val Ser Gly Ala Asp Val Val Ala His Ala His Arg Leu
Met Asn Phe Leu Tyr Val Ala Ser Pro Gly Arg Thr Leu Gly
SEQ ID NO: 4
   length: 522
   type: PRT
   source: Artificial Sequence
   note: Beta-subunit (522 aa) of wild-type Gl-7-ACA acylase protein
SEQ ID NO: 4 
Ser Asn Ser Trp Ala Val Ala Pro Gly Lys Thr Ala Asn Gly Asn Ala
Leu Leu Leu Gln Asn Pro His Leu Ser Trp Thr Thr Asp Tyr Phe Thr
Tyr Tyr Glu Ala His Leu Val Thr Pro Asp Phe Glu Ile Tyr Gly Ala
Thr Gln Ile Gly Leu Pro Val Ile Arg Phe Ala Phe Asn Gln Arg Met
Gly Ile Thr Asn Thr Val Asn Gly Met Val Gly Ala Thr Asn Tyr Arg
Leu Thr Leu Gln Asp Gly Gly Tyr Leu Tyr Asp Gly Gln Val Arg Pro
Phe Glu Arg Arg Gln Ala Ser Tyr Arg Leu Arg Gln Ala Asp Gly Thr
Thr Val Asp Lys Pro Leu Glu Ile Arg Ser Ser Val His Gly Pro Val
Phe Glu Arg Ala Asp Gly Thr Ala Val Ala Val Arg Val Ala Gly Leu
Asp Arg Pro Gly Met Leu Glu Gln Tyr Phe Asp Met Ile Thr Ala Asp
Ser Phe Asp Asp Tyr Glu Ala Ala Leu Ala Arg Met Gln Val Pro Thr
Phe Asn Ile Val Tyr Ala Asp Arg Glu Gly Thr Ile Asn Tyr Ser Phe
Asn Gly Val Ala Pro Lys Arg Ala Glu Gly Asp Ile Ala Phe Trp Gln
Gly Leu Val Pro Gly Asp Ser Ser Arg Tyr Leu Trp Thr Glu Thr His
Pro Leu Asp Asp Leu Pro Arg Val Thr Asn Pro Pro Gly Gly Phe Val
Gln Asn Ser Asn Asp Pro Pro Trp Thr Pro Thr Trp Pro Val Thr Tyr
Thr Pro Lys Asp Phe Pro Ser Tyr Leu Ala Pro Gln Thr Pro His Ser
Leu Arg Ala Gln Gln Ser Val Arg Leu Met Ser Glu Asn Asp Asp Leu
Thr Leu Glu Arg Phe Met Ala Leu Gln Leu Ser His Arg Ala Val Met
Ala Asp Arg Thr Leu Pro Asp Leu Ile Pro Ala Ala Leu Ile Asp Pro
Asp Pro Glu Val Gln Ala Ala Ala Arg Leu Leu Ala Ala Trp Asp Arg
Glu Phe Thr Ser Asp Ser Arg Ala Ala Leu Leu Phe Glu Glu Trp Ala
Arg Leu Phe Ala Gly Gln Asn Phe Ala Gly Gln Ala Gly Phe Ala Thr
Pro Trp Ser Leu Asp Lys Pro Val Ser Thr Pro Tyr Gly Val Arg Asp
Pro Lys Ala Ala Val Asp Gln Leu Arg Thr Ala Ile Ala Asn Thr Lys
Arg Lys Tyr Gly Ala Ile Asp Arg Pro Phe Gly Asp Ala Ser Arg Met
Ile Leu Asn Asp Val Asn Val Pro Gly Ala Ala Gly Tyr Gly Asn Leu
Gly Ser Phe Arg Val Phe Thr Trp Ser Asp Pro Asp Glu Asn Gly Val
Arg Thr Pro Val His Gly Glu Thr Trp Val Ala Met Ile Glu Phe Ser
Thr Pro Val Arg Ala Tyr Gly Leu Met Ser Tyr Gly Asn Ser Arg Gln
Pro Gly Thr Thr His Tyr Ser Asp Gln Ile Glu Arg Val Ser Arg Ala
Asp Phe Arg Glu Leu Leu Leu Arg Arg Glu Gln Val Glu Ala Ala Val
Gln Glu Arg Thr Pro Phe Asn Phe Lys Pro
SEQ ID NO: 5
   length: 11
   type: PRT
   source: Artificial Sequence
   note: Spacer (11 aa)
SEQ ID NO: 5
   Glu Gly Asp Pro Pro Asp Leu Ala Asp Gln Gly
SEQ ID NO: 6
   length: 692
   type: PRT
   source: Artificial Sequence
   note: PM2 mutant (692 aa) comprising wild-type alpha-subunit of Gl-7-ACA asylase from GK16, and mutant beta-subunit, and a spacer therebetween
SEQ ID NO: 6 
Met Glu Pro Thr Ser Thr Pro Gln Ala Pro Ile Ala Ala Tyr Lys Pro
Arg Ser Asn Glu Ile Leu Trp Asp Gly Tyr Gly Val Pro His Ile Tyr
Gly Val Asp Ala Pro Ser Ala Phe Tyr Gly Tyr Gly Trp Ala Gln Ala
Arg Ser His Gly Asp Asn Ile Leu Arg Leu Tyr Gly Glu Ala Arg Gly
Lys Gly Ala Glu Tyr Trp Gly Pro Asp Tyr Glu Gln Thr Thr Val Trp
Leu Leu Thr Asn Gly Val Pro Glu Arg Ala Gln Gln Trp Tyr Ala Gln
Gln Ser Pro Asp Phe Arg Ala Asn Leu Asp Ala Phe Ala Ala Gly Ile
Asn Ala Tyr Ala Gln Gln Asn Pro Asp Asp Ile Ser Pro Glu Val Arg
Gln Val Leu Pro Val Ser Gly Ala Asp Val Val Ala His Ala His Arg
Leu Met Asn Phe Leu Tyr Val Ala Ser Pro Gly Arg Thr Leu Gly Glu
Gly Asp Pro Pro Asp Leu Ala Asp Gln Gly Ser Asn Ser Trp Ala Val
Ala Pro Gly Lys Thr Ala Asn Gly Asn Ala Leu Leu Leu Gln Asn Pro
His Leu Ser Trp Thr Thr Asp Tyr Phe Thr Tyr Tyr Glu Ala His Leu
Val Thr Pro Asp Phe Glu Val Tyr Gly Ala Thr Gln Ile Gly Leu Pro
Val Ile Arg Val Ala Phe Asn Gln Arg Met Gly Ile Thr Asn Thr Val
Asn Gly Met Val Gly Ala Thr Asn Tyr Arg Leu Thr Leu Gln Asp Gly
Gly Tyr Leu Tyr Asp Gly Gln Val Arg Pro Phe Glu Arg Arg Gln Ala
Ser Tyr Arg Leu Arg Gln Ala Asp Gly Thr Thr Val Asp Lys Pro Leu
Glu Ile Arg Ser Ser Val His Gly Pro Val Phe Glu Arg Ala Asp Gly
Thr Ala Val Ala Val Arg Val Ala Gly Leu Asp Arg Pro Gly Met Leu
Glu Gln Thr Phe Asp Met Ile Thr Ala Asp Ser Phe Asp Asp Tyr Glu
Ala Ala Leu Ala Arg Met Gln Val Pro Thr Leu Asn Ile Val Tyr Ala
Asp Arg Glu Gly Thr Ile Asn Tyr Ser Phe Asn Gly Val Ala Pro Lys
Arg Ala Glu Gly Asp Ile Ala Phe Trp Gln Gly Leu Val Pro Gly Asp
Ser Ser Arg Tyr Leu Trp Thr Glu Thr His Pro Leu Asp Asp Leu Pro
Arg Val Thr Asn Pro Pro Gly Gly Phe Val Gln Asn Ser Asn Asp Pro
Pro Trp Thr Pro Thr Trp Pro Val Thr Tyr Thr Pro Lys Asp Phe Pro
Ser Tyr Leu Ala Pro Gln Thr Pro His Ser Leu Arg Ala Gln Gln Ser
Val Arg Leu Met Ser Glu Asn Asp Asp Leu Thr Leu Glu Arg Phe Met
Ala Leu Gln Leu Ser His Arg Ala Val Met Ala Asp Arg Thr Leu Pro
Asp Leu Ile Pro Ala Ala Leu Ile Asp Pro Asp Pro Glu Val Gln Ala
Ala Ala Arg Leu Leu Ala Ala Trp Asp Arg Glu Phe Thr Ser Asp Ser
Arg Ala Ala Leu Leu Phe Glu Glu Trp Ala Arg Leu Phe Ala Gly Gln
Asn Phe Ala Gly Gln Ala Gly Phe Ala Thr Pro Trp Ser Leu Asp Lys
Pro Val Ser Thr Pro Tyr Gly Leu Arg Asp Pro Lys Ala Ala Val Asp
Gln Leu Arg Thr Ala Ile Ala Asn Thr Lys Arg Lys Tyr Gly Ala Ile
Asp Arg Pro Phe Gly Asp Ala Ser Arg Met Ile Leu Asn Asp Val Asn
Val Pro Gly Ala Ala Gly Tyr Gly Asn Leu Gly Ser Phe Arg Val Phe
Thr Trp Ser Asp Pro Asp Glu Asn Gly Val Arg Thr Pro Val His Gly
Glu Thr Trp Val Ala Met Ile Glu Phe Ser Thr Pro Val Arg Ala Tyr
 
Gly Leu Met Ser Tyr Gly Asn Ser Arg Gln Pro Gly Thr Thr His Tyr
Ser Asp Gln Ile Glu Arg Val Ser Arg Ala Asp Phe Arg Glu Leu Leu
Leu Arg Arg Glu Gln Val Glu Ala Ala Val Gln Glu Arg Thr Pro Phe
Asn Phe Lys Pro
SEQ ID NO: 7
   length: 2079
   type: PRT
   source: Artificial Sequence
   note: Gene (2079 bp) encoding PM2 mutant
SEQ ID NO: 7 
Ala Thr Gly Gly Ala Ala Cys Cys Gly Ala Cys Cys Ala Gly Cys Ala
Cys Cys Cys Cys Gly Cys Ala Gly Gly Cys Thr Cys Cys Gly Ala Thr
Cys Gly Cys Thr Gly Cys Thr Thr Ala Thr Ala Ala Ala Cys Cys Gly
Cys Gly Thr Ala Gly Cys Ala Ala Cys Gly Ala Ala Ala Thr Cys Cys
Thr Gly Thr Gly Gly Gly Ala Thr Gly Gly Thr Thr Ala Thr Gly Gly
Thr Gly Thr Thr Cys Cys Gly Cys Ala Thr Ala Thr Cys Thr Ala Thr
Gly Gly Thr Gly Thr Thr Gly Ala Cys Gly Cys Thr Cys Cys Gly Ala
Gly Cys Gly Cys Thr Thr Thr Thr Thr Ala Thr Gly Gly Thr Thr Ala
Thr Gly Gly Cys Thr Gly Gly Gly Cys Thr Cys Ala Gly Gly Cys Thr
Cys Gly Thr Ala Gly Cys Cys Ala Thr Gly Gly Thr Gly Ala Thr Ala
Ala Cys Ala Thr Cys Cys Thr Gly Cys Gly Thr Cys Thr Gly Thr Ala
Thr Gly Gly Thr Gly Ala Ala Gly Cys Thr Cys Gly Thr Gly Gly Thr
Ala Ala Ala Gly Gly Cys Gly Cys Thr Gly Ala Ala Thr Ala Thr Thr
Gly Gly Gly Gly Thr Cys Cys Gly Gly Ala Thr Thr Ala Cys Gly Ala
Ala Cys Ala Gly Ala Cys Gly Ala Cys Cys Gly Thr Thr Thr Gly Gly
Cys Thr Gly Cys Thr Gly Ala Cys Cys Ala Ala Cys Gly Gly Thr Gly
Thr Thr Cys Cys Gly Gly Ala Ala Cys Gly Thr Gly Cys Thr Cys Ala
Gly Cys Ala Gly Thr Gly Gly Thr Ala Thr Gly Cys Thr Cys Ala Gly
Cys Ala Gly Ala Gly Cys Cys Cys Gly Gly Ala Thr Thr Thr Thr Cys
Gly Thr Gly Cys Thr Ala Ala Cys Cys Thr Gly Gly Ala Thr Gly Cys
Thr Thr Thr Thr Gly Cys Thr Gly Cys Thr Gly Gly Thr Ala Thr Cys
Ala Ala Cys Gly Cys Thr Thr Ala Thr Gly Cys Thr Cys Ala Gly Cys
Ala Gly Ala Ala Cys Cys Cys Gly Gly Ala Thr Gly Ala Thr Ala Thr
Cys Ala Gly Cys Cys Cys Gly Gly Ala Ala Gly Thr Thr Cys Gly Thr
Cys Ala Gly Gly Thr Thr Cys Thr Gly Cys Cys Gly Gly Thr Thr Ala
Gly Cys Gly Gly Thr Gly Cys Thr Gly Ala Thr Gly Thr Thr Gly Thr
Thr Gly Cys Thr Cys Ala Thr Gly Cys Thr Cys Ala Thr Cys Gly Thr
Cys Thr Gly Ala Thr Gly Ala Ala Cys Thr Thr Thr Cys Thr Gly Thr
Ala Thr Gly Thr Thr Gly Cys Thr Ala Gly Cys Cys Cys Gly Gly Gly
Thr Cys Gly Thr Ala Cys Cys Cys Thr Gly Gly Gly Thr Gly Ala Ala
Gly Gly Thr Gly Ala Thr Cys Cys Gly Cys Cys Gly Gly Ala Thr Cys
Thr Gly Gly Cys Thr Gly Ala Thr Cys Ala Gly Gly Gly Thr Ala Gly
Cys Ala Ala Cys Ala Gly Cys Thr Gly Gly Gly Cys Thr Gly Thr Thr
Gly Cys Thr Cys Cys Gly Gly Gly Thr Ala Ala Ala Ala Cys Cys Gly
Cys Thr Ala Ala Cys Gly Gly Thr Ala Ala Cys Gly Cys Thr Cys Thr
Gly Cys Thr Gly Cys Thr Gly Cys Ala Gly Ala Ala Cys Cys Cys Thr
Cys Ala Thr Cys Thr Gly Ala Gly Cys Thr Gly Gly Ala Cys Cys Ala
Cys Cys Gly Ala Thr Thr Ala Thr Thr Thr Ala Cys Cys Thr Ala
Thr Thr Ala Thr Gly Ala Ala Gly Cys Thr Cys Ala Thr Cys Thr Gly
Gly Thr Thr Ala Cys Cys Cys Cys Gly Gly Ala Thr Thr Thr Thr Gly
 
Ala Ala Gly Thr Gly Thr Ala Thr Gly Gly Thr Gly Cys Thr Ala Cys
Cys Cys Ala Gly Ala Thr Cys Gly Gly Thr Cys Thr Gly Cys Cys Gly
Gly Thr Thr Ala Thr Cys Cys Gly Thr Gly Thr Thr Gly Cys Thr Thr
Thr Thr Ala Ala Cys Cys Ala Gly Cys Gly Thr Ala Thr Gly Gly Gly
Thr Ala Thr Cys Ala Cys Cys Ala Ala Cys Ala Cys Cys Gly Thr Thr
Ala Ala Cys Gly Gly Thr Ala Thr Gly Gly Thr Thr Gly Gly Thr Gly
Cys Thr Ala Cys Cys Ala Ala Cys Thr Ala Thr Cys Gly Thr Cys Thr
Gly Ala Cys Cys Cys Thr Gly Cys Ala Gly Gly Ala Thr Gly Gly Thr
Gly Gly Thr Thr Ala Thr Cys Thr Gly Thr Ala Thr Gly Ala Thr Gly
Gly Thr Cys Ala Gly Gly Thr Thr Cys Gly Thr Cys Cys Gly Thr Thr
Thr Gly Ala Ala Cys Gly Thr Cys Gly Thr Cys Ala Gly Gly Cys Thr
Ala Gly Cys Thr Ala Thr Cys Gly Thr Cys Thr Gly Cys Gly Thr Cys
Ala Gly Gly Cys Thr Gly Ala Thr Gly Gly Thr Ala Cys Cys Ala Cys
Cys Gly Thr Thr Gly Ala Thr Ala Ala Ala Cys Cys Gly Cys Thr Gly
Gly Ala Ala Ala Thr Cys Cys Gly Thr Thr Cys Cys Ala Gly Cys Gly
Thr Thr Cys Ala Thr Gly Gly Thr Cys Cys Gly Gly Thr Thr Thr Thr
Thr Gly Ala Ala Cys Gly Thr Gly Cys Thr Gly Ala Thr Gly Gly Thr
Ala Cys Cys Gly Cys Thr Gly Thr Thr Gly Cys Thr Gly Thr Thr Cys
Gly Thr Gly Thr Thr Gly Cys Thr Gly Gly Thr Cys Thr Gly Gly Ala
Thr Cys Gly Thr Cys Cys Gly Gly Gly Thr Ala Thr Gly Cys Thr Gly
Gly Ala Ala Cys Ala Gly Ala Cys Thr Thr Thr Thr Gly Ala Thr Ala
Thr Gly Ala Thr Cys Ala Cys Cys Gly Cys Thr Gly Ala Thr Ala Gly
Cys Thr Thr Thr Gly Ala Thr Gly Ala Thr Thr Ala Thr Gly Ala Ala
Gly Cys Thr Gly Cys Thr Cys Thr Gly Gly Cys Thr Cys Gly Thr Ala
Thr Gly Cys Ala Gly Gly Thr Thr Cys Cys Gly Ala Cys Cys Thr Thr
Gly Ala Ala Cys Ala Thr Cys Gly Thr Thr Thr Ala Thr Gly Cys Thr
Gly Ala Thr Cys Gly Thr Gly Ala Ala Gly Gly Thr Ala Cys Cys Ala
Thr Cys Ala Ala Cys Thr Ala Thr Ala Gly Cys Thr Thr Thr Ala Ala
Cys Gly Gly Thr Gly Thr Thr Gly Cys Thr Cys Cys Gly Ala Ala Ala
Cys Gly Thr Gly Cys Thr Gly Ala Ala Gly Gly Thr Gly Ala Cys Ala
Thr Cys Gly Cys Thr Thr Thr Thr Thr Gly Gly Cys Ala Gly Gly Gly
Thr Cys Thr Gly Gly Thr Thr Cys Cys Gly Gly Gly Thr Gly Ala Thr
Ala Gly Cys Ala Gly Cys Cys Gly Thr Thr Ala Thr Cys Thr Gly Thr
Gly Gly Ala Cys Cys Gly Ala Ala Ala Cys Cys Cys Ala Thr Cys Cys
Gly Cys Thr Gly Gly Ala Thr Gly Ala Thr Cys Thr Gly Cys Cys Gly
Cys Gly Thr Gly Thr Thr Ala Cys Cys Ala Ala Cys Cys Cys Gly Cys
Cys Gly Gly Gly Thr Gly Gly Thr Thr Thr Thr Gly Thr Thr Cys Ala
Gly Ala Ala Cys Ala Gly Cys Ala Ala Cys Gly Ala Thr Cys Cys Gly
Cys Cys Gly Thr Gly Gly Ala Cys Cys Cys Cys Gly Ala Cys Cys Thr
Gly Gly Cys Cys Gly Gly Thr Thr Ala Cys Cys Thr Ala Thr Ala Cys
Cys Cys Cys Gly Ala Ala Ala Gly Ala Thr Thr Thr Thr Cys Cys Gly
Ala Gly Cys Thr Ala Thr Cys Thr Gly Gly Cys Thr Cys Cys Gly Cys
Ala Gly Ala Cys Cys Cys Cys Gly Cys Ala Thr Ala Gly Cys Cys Thr
Gly Cys Gly Thr Gly Cys Thr Cys Ala Gly Cys Ala Gly Ala Gly Cys
Gly Thr Thr Cys Gly Thr Cys Thr Gly Ala Thr Gly Ala Gly Cys Gly
Ala Ala Ala Ala Cys Gly Ala Thr Gly Ala Thr Cys Thr Gly Ala Cys
Cys Cys Thr Gly Gly Ala Ala Cys Gly Thr Thr Thr Thr Ala Thr Gly
Gly Cys Thr Cys Thr Gly Cys Ala Gly Cys Thr Gly Ala Gly Cys Cys
Ala Thr Cys Gly Thr Gly Cys Thr Gly Thr Thr Ala Thr Gly Gly Cys
Thr Gly Ala Thr Cys Gly Thr Ala Cys Cys Cys Thr Gly Cys Cys Gly
Gly Ala Thr Cys Thr Gly Ala Thr Cys Cys Cys Gly Gly Cys Thr Gly
 
Cys Thr Cys Thr Gly Ala Thr Cys Gly Ala Thr Cys Cys Gly Gly Ala
Thr Cys Cys Gly Gly Ala Ala Gly Thr Thr Cys Ala Gly Gly Cys Thr
Gly Cys Thr Gly Cys Thr Cys Gly Thr Cys Thr Gly Cys Thr Gly Gly
Cys Thr Gly Cys Gly Thr Gly Gly Gly Ala Thr Cys Gly Thr Gly Ala
Ala Thr Thr Thr Ala Cys Cys Ala Gly Cys Gly Ala Thr Ala Gly Cys
Cys Gly Thr Gly Cys Thr Gly Cys Thr Cys Thr Gly Cys Thr Gly Thr
Thr Thr Gly Ala Ala Gly Ala Ala Thr Gly Gly Gly Cys Thr Cys Gly
Thr Cys Thr Gly Thr Thr Thr Gly Cys Thr Gly Gly Thr Cys Ala Gly
Ala Ala Cys Thr Thr Thr Gly Cys Thr Gly Gly Thr Cys Ala Gly Gly
Cys Thr Gly Gly Cys Thr Thr Thr Gly Cys Thr Ala Cys Cys Cys Cys
Gly Thr Gly Gly Ala Gly Cys Cys Thr Gly Gly Ala Thr Ala Ala Ala
Cys Cys Gly Gly Thr Thr Ala Gly Cys Ala Cys Cys Cys Cys Gly Thr
Ala Thr Gly Gly Thr Cys Thr Gly Cys Gly Thr Gly Ala Thr Cys Cys
Gly Ala Ala Ala Gly Cys Thr Gly Cys Thr Gly Thr Thr Gly Ala Thr
Cys Ala Gly Cys Thr Gly Cys Gly Thr Ala Cys Cys Gly Cys Thr Ala
Thr Cys Gly Cys Thr Ala Ala Cys Ala Cys Cys Ala Ala Ala Cys Gly
Thr Ala Ala Ala Thr Ala Thr Gly Gly Thr Gly Cys Thr Ala Thr Cys
Gly Ala Thr Cys Gly Thr Cys Cys Gly Thr Thr Thr Gly Gly Thr Gly
Ala Thr Gly Cys Thr Ala Gly Cys Cys Gly Thr Ala Thr Gly Ala Thr
Cys Cys Thr Gly Ala Ala Cys Gly Ala Thr Gly Thr Thr Ala Ala Cys
Gly Thr Thr Cys Cys Gly Gly Gly Thr Gly Cys Thr Gly Cys Thr Gly
Gly Thr Thr Ala Thr Gly Gly Thr Ala Ala Cys Cys Thr Gly Gly Gly
Thr Ala Gly Cys Thr Thr Thr Cys Gly Thr Gly Thr Thr Thr Thr Thr
Ala Cys Cys Thr Gly Gly Ala Gly Cys Gly Ala Thr Cys Cys Gly Gly
Ala Thr Gly Ala Ala Ala Ala Cys Gly Gly Thr Gly Thr Gly Cys Gly
Thr Ala Cys Cys Cys Cys Gly Gly Thr Thr Cys Ala Thr Gly Gly Thr
Gly Ala Ala Ala Cys Cys Thr Gly Gly Gly Thr Thr Gly Cys Thr Ala
Thr Gly Ala Thr Cys Gly Ala Ala Thr Thr Thr Ala Gly Cys Ala Cys
Cys Cys Cys Gly Gly Thr Thr Cys Gly Thr Gly Cys Thr Thr Ala Thr
Gly Gly Thr Cys Thr Gly Ala Thr Gly Ala Gly Cys Thr Ala Thr Gly
Gly Thr Ala Ala Cys Ala Gly Cys Cys Gly Thr Cys Ala Gly Cys Cys
Gly Gly Gly Thr Ala Cys Cys Ala Cys Cys Cys Ala Thr Thr Ala Thr
Ala Gly Cys Gly Ala Thr Cys Ala Gly Ala Thr Cys Gly Ala Ala Cys
Gly Thr Gly Thr Thr Ala Gly Cys Cys Gly Thr Gly Cys Thr Gly Ala
Thr Thr Thr Thr Cys Gly Thr Gly Ala Ala Cys Thr Gly Cys Thr Gly
Cys Thr Gly Cys Gly Thr Cys Gly Thr Gly Ala Ala Cys Ala Gly Gly
Gly Gly Ala Ala Cys Gly Thr Ala Cys Cys Cys Cys Gly Thr Thr Thr
Ala Ala Cys Thr Thr Thr Ala Ala Ala Cys Cys Gly Thr Gly Ala
SEQ ID NO: 8
   length: 522
   type: PRT
   source: Artificial Sequence
   note: Beta-subunit of mutant PM2 protein
SEQ ID NO: 8 
Ser Asn Ser Trp Ala Val Ala Pro Gly Lys Thr Ala Asn Gly Asn Ala
Leu Leu Leu Gln Asn Pro His Leu Ser Trp Thr Thr Asp Tyr Phe Thr
Tyr Tyr Glu Ala His Leu Val Thr Pro Asp Phe Glu Val Tyr Gly Ala
Thr Gln Ile Gly Leu Pro Val Ile Arg Val Ala Phe Asn Gln Arg Met
Gly Ile Thr Asn Thr Val Asn Gly Met Val Gly Ala Thr Asn Tyr Arg
Leu Thr Leu Gln Asp Gly Gly Tyr Leu Tyr Asp Gly Gln Val Arg Pro
 
Phe Glu Arg Arg Gln Ala Ser Tyr Arg Leu Arg Gln Ala Asp Gly Thr
Thr Val Asp Lys Pro Leu Glu Ile Arg Ser Ser Val His Gly Pro Val
Phe Glu Arg Ala Asp Gly Thr Ala Val Ala Val Arg Val Ala Gly Leu
Asp Arg Pro Gly Met Leu Glu Gln Thr Phe Asp Met Ile Thr Ala Asp
Ser Phe Asp Asp Tyr Glu Ala Ala Leu Ala Arg Met Gln Val Pro Thr
Leu Asn Ile Val Tyr Ala Asp Arg Glu Gly Thr Ile Asn Tyr Ser Phe
Asn Gly Val Ala Pro Lys Arg Ala Glu Gly Asp Ile Ala Phe Trp Gln
Gly Leu Val Pro Gly Asp Ser Ser Arg Tyr Leu Trp Thr Glu Thr His
Pro Leu Asp Asp Leu Pro Arg Val Thr Asn Pro Pro Gly Gly Phe Val
Gln Asn Ser Asn Asp Pro Pro Trp Thr Pro Thr Trp Pro Val Thr Tyr
Thr Pro Lys Asp Phe Pro Ser Tyr Leu Ala Pro Gln Thr Pro His Ser
Leu Arg Ala Gln Gln Ser Val Arg Leu Met Ser Glu Asn Asp Asp Leu
Thr Leu Glu Arg Phe Met Ala Leu Gln Leu Ser His Arg Ala Val Met
Ala Asp Arg Thr Leu Pro Asp Leu Ile Pro Ala Ala Leu Ile Asp Pro
Asp Pro Glu Val Gln Ala Ala Ala Arg Leu Leu Ala Ala Trp Asp Arg
Glu Phe Thr Ser Asp Ser Arg Ala Ala Leu Leu Phe Glu Glu Trp Ala
Arg Leu Phe Ala Gly Gln Asn Phe Ala Gly Gln Ala Gly Phe Ala Thr
Pro Trp Ser Leu Asp Lys Pro Val Ser Thr Pro Tyr Gly Leu Arg Asp
Pro Lys Ala Ala Val Asp Gln Leu Arg Thr Ala Ile Ala Asn Thr Lys
Arg Lys Tyr Gly Ala Ile Asp Arg Pro Phe Gly Asp Ala Ser Arg Met
Ile Leu Asn Asp Val Asn Val Pro Gly Ala Ala Gly Tyr Gly Asn Leu
Gly Ser Phe Arg Val Phe Thr Trp Ser Asp Pro Asp Glu Asn Gly Val
Arg Thr Pro Val His Gly Glu Thr Trp Val Ala Met Ile Glu Phe Ser
Thr Pro Val Arg Ala Tyr Gly Leu Met Ser Tyr Gly Asn Ser Arg Gln
Pro Gly Thr Thr His Tyr Ser Asp Gln Ile Glu Arg Val Ser Arg Ala
Asp Phe Arg Glu Leu Leu Leu Arg Arg Glu Gln Val Glu Ala Ala Val
Gln Glu Arg Thr Pro Phe Asn Phe Lys Pro
SEQ ID NO: 9
   length: 692
   type: PRT
   source: Artificial Sequence
   note: mutant CSH17 protein
SEQ ID NO: 9 
Met Glu Pro Thr Ser Thr Pro Gln Ala Pro Ile Asn Ala Tyr Lys Pro
Arg Ser Asn Glu Ile Leu Trp Asp Asp Tyr Gly Val Pro His Ile Tyr
Gly Val Asp Ala Pro Ser Ala Phe Tyr Gly Tyr Gly Trp Ala Gln Ala
Arg Ser His Gly Asp Asn Ile Leu Arg Leu Tyr Gly Glu Ala Arg Gly
Lys Gly Ala Glu Tyr Trp Gly Pro Asp Tyr Glu Gln Thr Thr Val Trp
Leu Leu Thr Asn Gly Val Pro Glu Arg Ala Gln Gln Trp Tyr Ala Gln
Gln Ser Pro Asp Phe Arg Ala Asn Leu Asp Ala Phe Ala Ala Gly Ile
Asn Ala Tyr Ala Gln Gln Asn Pro Asp Asp Ile Ser Pro Glu Val Arg
Gln Val Leu Pro Val Ser Gly Ala Asp Val Val Ala His Ala His Arg
Leu Met Asn Phe Leu Tyr Val Ala Ser Pro Gly Arg Thr Leu Gly Glu
Gly Asp Pro Pro Asp Leu Ala Asp Gln Gly Ser Asn Ser Trp Ala Val
Ala Pro Gly Lys Thr Ala Asn Gly Asn Ala Leu Leu Leu Gln Asn Pro
His Leu Ser Trp Thr Thr Asp Tyr Phe Thr Tyr Tyr Glu Ala His Leu
Val Thr Pro Asp Phe Glu Val Tyr Gly Ala Thr Gln Ile Gly Leu Pro
Val Ile Arg Val Ala Phe Asn Gln Arg Met Gly Ile Thr Asn Thr Val
Asn Gly Met Val Gly Ala Thr Asn Tyr Arg Leu Thr Leu Gln Asp Gly
Gly Tyr Leu Tyr Asp Gly Gln Val Arg Pro Phe Glu Arg Arg Gln Ala
 
Ser Tyr Arg Leu Arg Gln Ala Asp Gly Thr Thr Val Asp Lys Pro Leu
Glu Ile Arg Ser Ser Val His Gly Pro Val Phe Glu Arg Ala Asp Gly
Thr Thr Val Ala Val Arg Val Ala Gly Leu Asp Arg Pro Gly Met Leu
Glu Gln Thr Phe Asp Met Ile Thr Ala Asp Ser Phe Asp Asp Tyr Glu
Ala Ala Leu Ala Arg Met Gln Val Pro Thr Leu Asn Tyr Val Tyr Ala
Asp Arg Glu Gly Thr Ile Asn Tyr Ser Phe Asn Gly Val Ala Pro Lys
Arg Ala Glu Gly Asp Ile Ala Phe Trp Gln Gly Leu Val Pro Gly Asp
Ser Ser Arg Tyr Leu Trp Thr Glu Thr His Pro Leu Asp Asp Leu Pro
Arg Val Thr Asn Pro Pro Gly Gly Phe Val Gln Asn Ser Asn Asp Pro
Pro Trp Thr Pro Thr Trp Pro Val Thr Tyr Thr Pro Lys Asp Phe Pro
Ser Tyr Leu Ala Pro Gln Thr Pro His Ser Leu Arg Ala Gln Gln Ser
Val Arg Leu Met Ser Glu Asn Asp Asp Leu Thr Leu Glu Arg Phe Met
Ala Leu Gln Leu Ser His Arg Ala Val Met Ala Asp Arg Thr Leu Pro
Asp Leu Ile Pro Ala Ala Leu Ile Asp Pro Asp Pro Glu Val Gln Ala
Ala Ala Arg Leu Leu Ala Ala Trp Asp Arg Glu Phe Thr Ser Asp Ser
Arg Ala Ala Leu Leu Phe Glu Glu Trp Ala Arg Leu Phe Ala Gly Gln
Asn Phe Ala Gly Gln Ala Gly Phe Ala Thr Pro Trp Ser Leu Asp Lys
Pro Val Ser Thr Pro Tyr Gly Leu Arg Asp Pro Lys Ala Ala Val Asp
Gln Leu Arg Thr Ala Ile Ala Asn Thr Lys Arg Lys Tyr Gly Ala Ile
Asp Arg Pro Phe Gly Asp Ala Ser Arg Met Ile Leu Asn Asp Val Asn
Val Pro Gly Ala Ala Gly Tyr Gly Asn Leu Gly Ser Phe Arg Val Phe
Thr Trp Ser Asp Pro Asp Glu Asn Gly Val Arg Thr Pro Val His Gly
Glu Thr Trp Val Ala Met Ile Glu Phe Ser Thr Pro Val Arg Ala Tyr
Gly Leu Met Ser Tyr Gly Asn Ser Arg Gln Pro Gly Thr Thr His Tyr
Ser Asp Gln Ile Glu Arg Val Ser Arg Ala Asp Phe Arg Glu Leu Leu
Leu Arg Arg Glu Gln Val Glu Ala Ala Val Gln Glu Arg Thr Pro Phe
Asn Phe Lys Pro
SEQ ID NO: 10
   length: 158
   type: PRT
   source: Artificial Sequence
   note: Alpha-subunit of mutant CSH17 protein
SEQ ID NO: 10 
Glu Pro Thr Ser Thr Pro Gln Ala Pro Ile Asn Ala Tyr Lys Pro Arg
Ser Asn Glu Ile Leu Trp Asp Asp Tyr Gly Val Pro His Ile Tyr Gly
Val Asp Ala Pro Ser Ala Phe Tyr Gly Tyr Gly Trp Ala Gln Ala Arg
Ser His Gly Asp Asn Ile Leu Arg Leu Tyr Gly Glu Ala Arg Gly Lys
Gly Ala Glu Tyr Trp Gly Pro Asp Tyr Glu Gln Thr Thr Val Trp Leu
Leu Thr Asn Gly Val Pro Glu Arg Ala Gln Gln Trp Tyr Ala Gln Gln
Ser Pro Asp Phe Arg Ala Asn Leu Asp Ala Phe Ala Ala Gly Ile Asn
Ala Tyr Ala Gln Gln Asn Pro Asp Asp Ile Ser Pro Glu Val Arg Gln
Val Leu Pro Val Ser Gly Ala Asp Val Val Ala His Ala His Arg Leu
Met Asn Phe Leu Tyr Val Ala Ser Pro Gly Arg Thr Leu Gly
SEQ ID NO: [?]
SEQ ID NO: 11
   length: 522
   type: PRT
   source: Artificial Sequence
   note: Beta-subunit of mutant CSH17 protein
SEQ ID NO: 11 
Ser Asn Ser Trp Ala Val Ala Pro Gly Lys Thr Ala Asn Gly Asn Ala
Leu Leu Leu Gln Asn Pro His Leu Ser Trp Thr Thr Asp Tyr Phe Thr
Tyr Tyr Glu Ala His Leu Val Thr Pro Asp Phe Glu Val Tyr Gly Ala
Thr Gln Ile Gly Leu Pro Val Ile Arg Val Ala Phe Asn Gln Arg Met
Gly Ile Thr Asn Thr Val Asn Gly Met Val Gly Ala Thr Asn Tyr Arg
Leu Thr Leu Gln Asp Gly Gly Tyr Leu Tyr Asp Gly Gln Val Arg Pro
Phe Glu Arg Arg Gln Ala Ser Tyr Arg Leu Arg Gln Ala Asp Gly Thr
Thr Val Asp Lys Pro Leu Glu Ile Arg Ser Ser Val His Gly Pro Val
Phe Glu Arg Ala Asp Gly Thr Thr Val Ala Val Arg Val Ala Gly Leu
Asp Arg Pro Gly Met Leu Glu Gln Thr Phe Asp Met Ile Thr Ala Asp
Ser Phe Asp Asp Tyr Glu Ala Ala Leu Ala Arg Met Gln Val Pro Thr
Leu Asn Tyr Val Tyr Ala Asp Arg Glu Gly Thr Ile Asn Tyr Ser Phe
Asn Gly Val Ala Pro Lys Arg Ala Glu Gly Asp Ile Ala Phe Trp Gln
Gly Leu Val Pro Gly Asp Ser Ser Arg Tyr Leu Trp Thr Glu Thr His
Pro Leu Asp Asp Leu Pro Arg Val Thr Asn Pro Pro Gly Gly Phe Val
Gln Asn Ser Asn Asp Pro Pro Trp Thr Pro Thr Trp Pro Val Thr Tyr
Thr Pro Lys Asp Phe Pro Ser Tyr Leu Ala Pro Gln Thr Pro His Ser
Leu Arg Ala Gln Gln Ser Val Arg Leu Met Ser Glu Asn Asp Asp Leu
Thr Leu Glu Arg Phe Met Ala Leu Gln Leu Ser His Arg Ala Val Met
Ala Asp Arg Thr Leu Pro Asp Leu Ile Pro Ala Ala Leu Ile Asp Pro
Asp Pro Glu Val Gln Ala Ala Ala Arg Leu Leu Ala Ala Trp Asp Arg
Glu Phe Thr Ser Asp Ser Arg Ala Ala Leu Leu Phe Glu Glu Trp Ala
Arg Leu Phe Ala Gly Gln Asn Phe Ala Gly Gln Ala Gly Phe Ala Thr
Pro Trp Ser Leu Asp Lys Pro Val Ser Thr Pro Tyr Gly Leu Arg Asp
Pro Lys Ala Ala Val Asp Gln Leu Arg Thr Ala Ile Ala Asn Thr Lys
Arg Lys Tyr Gly Ala Ile Asp Arg Pro Phe Gly Asp Ala Ser Arg Met
Ile Leu Asn Asp Val Asn Val Pro Gly Ala Ala Gly Tyr Gly Asn Leu
Gly Ser Phe Arg Val Phe Thr Trp Ser Asp Pro Asp Glu Asn Gly Val
Arg Thr Pro Val His Gly Glu Thr Trp Val Ala Met Ile Glu Phe Ser
Thr Pro Val Arg Ala Tyr Gly Leu Met Ser Tyr Gly Asn Ser Arg Gln
Pro Gly Thr Thr His Tyr Ser Asp Gln Ile Glu Arg Val Ser Arg Ala
Asp Phe Arg Glu Leu Leu Leu Arg Arg Glu Gln Val Glu Ala Ala Val
Gln Glu Arg Thr Pro Phe Asn Phe Lys Pro
SEQ ID NO: 12
   length: 692
   type: PRT
   source: Artificial Sequence
   note: mutant TEP11 protein
SEQ ID NO: 12 
Met Glu Pro Thr Ser Thr Pro Gln Ala Pro Ile Asn Ala Tyr Lys Pro
Arg Ser Asn Glu Ile Leu Trp Asp Asp Tyr Gly Val Pro His Ile Tyr
Gly Val Asp Ala Pro Ser Ala Phe Tyr Gly Tyr Gly Trp Ala Gln Ala
Arg Ser His Gly Asp Asn Ile Leu Arg Leu Tyr Gly Glu Ala Arg Gly
Lys Gly Ala Glu Tyr Trp Gly Pro Asp Tyr Glu Gln Thr Thr Val Trp
Leu Leu Thr Asn Gly Val Pro Glu Arg Ala Gln Gln Trp Tyr Ala Gln
Gln Ser Pro Asp Phe Arg Ala Asn Leu Asp Ala Phe Ala Ala Gly Ile
Asn Ala Tyr Ala Gln Gln Asn Pro Asp Asp Ile Ser Pro Glu Val Arg
Gln Val Leu Pro Val Ser Gly Ala Asp Val Val Ala His Ala His Arg
Leu Met Asn Phe Leu Tyr Val Ala Ser Pro Gly Arg Thr Leu Gly Glu
Gly Asp Pro Pro Asp Leu Ala Asp Gln Gly Ser Asn Ser Trp Ala Val
 
Ala Pro Gly Lys Thr Ala Asn Gly Asn Ala Leu Leu Leu Gln Asn Pro
His Leu Ser Trp Thr Thr Asp Tyr Phe Thr Tyr Tyr Glu Ala His Leu
Val Thr Pro Asp Phe Glu Val Tyr Gly Ala Thr Gln Ile Gly Leu Pro
Val Ile Arg Val Ala Phe Asn Gln Arg Met Gly Ile Thr Asn Thr Val
Asn Gly Met Val Gly Ala Thr Asn Tyr Arg Leu Thr Leu Gln Asp Gly
Gly Tyr Leu Tyr Asp Gly Gln Val Arg Pro Phe Glu Arg Arg Gln Ala
Ser Tyr Arg Leu Arg Gln Ala Asp Gly Thr Thr Val Asp Lys Pro Leu
Glu Ile Arg Ser Ser Val His Gly Pro Val Phe Glu Arg Ala Asp Gly
Thr Thr Val Ala Val Arg Val Ala Gly Leu Asp Arg Pro Gly Met Leu
Glu Gln Thr Phe Asp Met Ile Thr Ala Asp Ser Phe Asp Asp Tyr Glu
Ala Ala Leu Ala Arg Met Gln Val Pro Thr Leu Asn Tyr Val Tyr Ala
Asp Arg Glu Gly Thr Ile Asn Tyr Ser Phe Asn Gly Val Ala Pro Lys
Arg Ala Glu Gly Asp Ile Ala Phe Trp Gln Gly Leu Val Pro Gly Asp
Ser Ser Arg Tyr Leu Trp Thr Glu Thr His Pro Leu Asp Asp Leu Pro
Arg Val Thr Asn Pro Pro Gly Gly Phe Val Gln Asn Ser Asn Asp Pro
Pro Trp Thr Pro Thr Trp Pro Val Thr Tyr Thr Pro Lys Asp Phe Pro
Ser Tyr Leu Ala Pro Gln Thr Pro His Ser Leu Arg Ala Gln Gln Ser
Val Arg Leu Met Ser Glu Asn Asp Asp Leu Thr Leu Glu Arg Phe Met
Ala Leu Gln Leu Ser His Arg Ala Val Met Ala Asp Arg Thr Leu Pro
Asp Leu Ile Pro Ala Ala Leu Ile Asp Pro Asp Pro Glu Val Gln Ala
Ala Ala Arg Leu Leu Ala Ala Trp Asp Arg Glu Phe Thr Ser Asp Ser
Arg Ala Ala Leu Leu Phe Glu Glu Trp Ala Arg Leu Phe Ala Gly Gln
Asn Phe Ala Gly Gln Ala Gly Phe Ala Thr Pro Trp Ser Leu Asp Lys
Pro Val Ser Thr Pro Tyr Gly Leu Arg Asp Pro Lys Ala Ala Val Asp
Gln Leu Arg Thr Ala Ile Ala Asn Thr Lys Arg Lys Tyr Gly Ala Ile
Asp Arg Pro Phe Gly Asp Ala Ser Arg Met Ile Leu Asn Asp Val Asn
Val Pro Gly Ala Ala Gly Tyr Gly Asn Leu Gly Ser Phe Arg Val Phe
Thr Trp Ser Asp Pro Asp Glu Asn Gly Val Arg Thr Pro Val Thr Gly
Glu Thr Trp Val Ala Met Ile Glu Phe Ser Thr Pro Val Arg Ala Tyr
Gly Leu Met Ser Tyr Gly Asn Ser Arg Gln Pro Gly Thr Thr His Tyr
Ser Asp Gln Ile Glu Arg Val Ser Arg Ala Asp Phe Arg Glu Leu Leu
Leu Arg Arg Glu Gln Val Glu Ala Ala Val Gln Glu Arg Thr Pro Phe
Asn Phe Lys Pro
SEQ ID NO: 13
   length: 158
   type: PRT
   source: Artificial Sequence
   note: Alpha-subunit of mutant TEP11 protein
SEQ ID NO: 13 
Glu Pro Thr Ser Thr Pro Gln Ala Pro Ile Asn Ala Tyr Lys Pro Arg
Ser Asn Glu Ile Leu Trp Asp Asp Tyr Gly Val Pro His Ile Tyr Gly
Val Asp Ala Pro Ser Ala Phe Tyr Gly Tyr Gly Trp Ala Gln Ala Arg
Ser His Gly Asp Asn Ile Leu Arg Leu Tyr Gly Glu Ala Arg Gly Lys
Gly Ala Glu Tyr Trp Gly Pro Asp Tyr Glu Gln Thr Thr Val Trp Leu
Leu Thr Asn Gly Val Pro Glu Arg Ala Gln Gln Trp Tyr Ala Gln Gln
Ser Pro Asp Phe Arg Ala Asn Leu Asp Ala Phe Ala Ala Gly Ile Asn
Ala Tyr Ala Gln Gln Asn Pro Asp Asp Ile Ser Pro Glu Val Arg Gln
Val Leu Pro Val Ser Gly Ala Asp Val Val Ala His Ala His Arg Leu
Met Asn Phe Leu Tyr Val Ala Ser Pro Gly Arg Thr Leu Gly
SEQ ID NO: 14
   length: 522
   type: PRT
   source: Artificial Sequence
   note: Beta-subunit of mutant TEP11
SEQ ID NO: 14 
Ser Asn Ser Trp Ala Val Ala Pro Gly Lys Thr Ala Asn Gly Asn Ala
Leu Leu Leu Gln Asn Pro His Leu Ser Trp Thr Thr Asp Tyr Phe Thr
Tyr Tyr Glu Ala His Leu Val Thr Pro Asp Phe Glu Val Tyr Gly Ala
Thr Gln Ile Gly Leu Pro Val Ile Arg Val Ala Phe Asn Gln Arg Met
Gly Ile Thr Asn Thr Val Asn Gly Met Val Gly Ala Thr Asn Tyr Arg
Leu Thr Leu Gln Asp Gly Gly Tyr Leu Tyr Asp Gly Gln Val Arg Pro
Phe Glu Arg Arg Gln Ala Ser Tyr Arg Leu Arg Gln Ala Asp Gly Thr
Thr Val Asp Lys Pro Leu Glu Ile Arg Ser Ser Val His Gly Pro Val
Phe Glu Arg Ala Asp Gly Thr Thr Val Ala Val Arg Val Ala Gly Leu
Asp Arg Pro Gly Met Leu Glu Gln Thr Phe Asp Met Ile Thr Ala Asp
Ser Phe Asp Asp Tyr Glu Ala Ala Leu Ala Arg Met Gln Val Pro Thr
Leu Asn Tyr Val Tyr Ala Asp Arg Glu Gly Thr Ile Asn Tyr Ser Phe
Asn Gly Val Ala Pro Lys Arg Ala Glu Gly Asp Ile Ala Phe Trp Gln
Gly Leu Val Pro Gly Asp Ser Ser Arg Tyr Leu Trp Thr Glu Thr His
Pro Leu Asp Asp Leu Pro Arg Val Thr Asn Pro Pro Gly Gly Phe Val
Gln Asn Ser Asn Asp Pro Pro Trp Thr Pro Thr Trp Pro Val Thr Tyr
Thr Pro Lys Asp Phe Pro Ser Tyr Leu Ala Pro Gln Thr Pro His Ser
Leu Arg Ala Gln Gln Ser Val Arg Leu Met Ser Glu Asn Asp Asp Leu
Thr Leu Glu Arg Phe Met Ala Leu Gln Leu Ser His Arg Ala Val Met
Ala Asp Arg Thr Leu Pro Asp Leu Ile Pro Ala Ala Leu Ile Asp Pro
Asp Pro Glu Val Gln Ala Ala Ala Arg Leu Leu Ala Ala Trp Asp Arg
Glu Phe Thr Ser Asp Ser Arg Ala Ala Leu Leu Phe Glu Glu Trp Ala
Arg Leu Phe Ala Gly Gln Asn Phe Ala Gly Gln Ala Gly Phe Ala Thr
Pro Trp Ser Leu Asp Lys Pro Val Ser Thr Pro Tyr Gly Leu Arg Asp
Pro Lys Ala Ala Val Asp Gln Leu Arg Thr Ala Ile Ala Asn Thr Lys
Arg Lys Tyr Gly Ala Ile Asp Arg Pro Phe Gly Asp Ala Ser Arg Met
Ile Leu Asn Asp Val Asn Val Pro Gly Ala Ala Gly Tyr Gly Asn Leu
Gly Ser Phe Arg Val Phe Thr Trp Ser Asp Pro Asp Glu Asn Gly Val
Arg Thr Pro Val Thr Gly Glu Thr Trp Val Ala Met Ile Glu Phe Ser
Thr Pro Val Arg Ala Tyr Gly Leu Met Ser Tyr Gly Asn Ser Arg Gln
Pro Gly Thr Thr His Tyr Ser Asp Gln Ile Glu Arg Val Ser Arg Ala
Asp Phe Arg Glu Leu Leu Leu Arg Arg Glu Gln Val Glu Ala Ala Val
Gln Glu Arg Thr Pro Phe Asn Phe Lys Pro
SEQ ID NO: 15
   length: 20
   type: DNA
   source: Artificial Sequence
   note: T3 primer
SEQ ID NO: 15
   aattaaccct cactaaaggg 20
SEQ ID NO: 16
   length: 20
   type: DNA
   source: Artificial Sequence
   note: T7 primer
SEQ ID NO: 16
   taatacgact cactataggg 20
SEQ ID NO: 17
   length: 20
   type: DNA
   source: Artificial Sequence
   note: M13-F primer
SEQ ID NO: 17
   ttgtaaaacg acggccagtg 20
SEQ ID NO: 18
   length: 19
   type: DNA
   source: Artificial Sequence
   note: M13-R primer
SEQ ID NO: 18
   ggaaacagct atgaccatg 19
SEQ ID NO: 19
   length: 30
   type: DNA
   source: Artificial Sequence
   note: SH24a-F primer (non-opti)
SEQ ID NO: 19
   gaaatcctgt gggatgryta tggtgttccg 30
SEQ ID NO: 20
   length: 30
   type: DNA
   source: Artificial Sequence
   note: SH24a-R primer
SEQ ID NO: 20
   cggaacacca tarycatccc acaggatttc 30
SEQ ID NO: 21
   length: 33
   type: DNA
   source: Artificial Sequence
   note: SH179b-F primer
SEQ ID NO: 21
   gttccgaccc ttaacwwygt ttatgetgat cgt 33
SEQ ID NO: 22
   length: 33
   type: DNA
   source: Artificial Sequence
   note: SH179b-R primer
SEQ ID NO: 22
   acgatcagca taaacrwwgt taagggtcgg aac 33
SEQ ID NO: 23
   length: 34
   type: DNA
   source: Artificial Sequence
   note: SM7_F primer
SEQ ID NO: 23
   gttccgacct ttaactatgt ttatgetgat cgtg 34
SEQ ID NO: 24
   length: 34
   type: DNA
   source: Artificial Sequence
   note: SM7_R primer
SEQ ID NO: 24
   cacgatcagc ataaacatag ttaaaggtcg gaac 34
SEQ ID NO: 25
   length: 36
   type: DNA
   source: Artificial Sequence
   note: SM10_F primer
SEQ ID NO: 25
   gaaatcctgt gggatgacta tggtgttccg catate 36
SEQ ID NO: 26
   length: 36
   type: DNA
   source: Artificial Sequence
   note: SM10_R primer
SEQ ID NO: 26
   gatatgcgga acaccatagt catcccacag gatttc 36
SEQ ID NO: 27
   length: 34
   type: DNA
   source: Artificial Sequence
   note: SM25_F primer
SEQ ID NO: 27
   cgtgctgatg gtaccaccgt tgctgttcgt gttg 34
SEQ ID NO: 28
   length: 34
   type: DNA
   source: Artificial Sequence
   note: SM25_R primer
SEQ ID NO: 28
   caacacgaac agcaacggtg gtaccatcag cacg 34
SEQ ID NO: 29
   length: 36
   type: DNA
   source: Artificial Sequence
   note: SM43_F primer
SEQ ID NO: 29
   ccgcaggctc cgatcaatgc ttataaaccg cgtage 36
SEQ ID NO: 30
   length: 36
   type: DNA
   source: Artificial Sequence
   note: SM43_R primer
SEQ ID NO: 30
   gctacgcggt ttataagcat tgatcggagc ctgcgg 36
SEQ ID NO: 31
   length: 35
   type: DNA
   source: Artificial Sequence
   note: SM52_F primer
SEQ ID NO: 31
   gtgcgtaccc cggttacggg tgaaacctgg gttgc 35
SEQ ID NO: 32
   length: 35
   type: DNA
   source: Artificial Sequence
   note: SM52_R primer
SEQ ID NO: 32
   gcaacccagg tttcacccgt aaccggggta cgcac 35

## Claims

1. A mutant cephalosporin C (CPC) acylase, derived from a CPC acylase,
wherein the mutant CPC acylase comprises an alpha-subunit of SEQ ID NO: 3 and a beta-subunit of SEQ ID NO: 4 and all the following substitutions:
substitution of alanine at position 11 in the alpha-subunit of (A11α) with asparagine (N);
substitution of glycine at position 24 in the alpha-subunit of (G24α) with aspartic acid (D);
substitution of alanine at position 136 in the beta-subunit of (A136β) with threonine (T); and
substitution of isoleucine at position 179 in the beta-subunit of (I179β) with tyrosine (Y).

2. The mutant CPC acylase of claim 1, wherein the mutant CPC acylase further comprises a substitution of histidine at position 453 in the beta-subunit of SEQ ID NO: 4 with threonine (T).

3. The mutant CPC acylase of claim 1 or 2, wherein the mutant CPC acylase further comprises an additional substitution of at least one amino acid selected from the group consisting of:
substitution of I45ß with valine (V) (I45ßV);
substitution of F58ß with valine(V) (F58ßV);
substitution of Y153ß with threonine (T) (Y153ßT);
substitution of F177ß with leucine (L) (F177ßL); and
substitution of V382ß with leucine (L) (V382ßL),
in the beta-subunit of SEQ ID NO: 4.

4. The mutant CPC acylase of claim 3, wherein the additional substitution comprises all of the following substitutions:
substitution of I45ß with valine (V) (I45ßV);
substitution of F58ß with valine(V) (F58βV);
substitution of Y153β with threonine (T) (Y153βT);
substitution of F177ß with leucine (L) (F177ßL); and
substitution of V382ß with leucine (L) (V382ßL),
in the beta-subunit of SEQ ID NO: 4.

5. The mutant CPC acylase of claim 3, wherein the mutant CPC acylase contains the amino acid sequence of SEQ ID NO: 9 or SEQ ID NO: 12.

6. A nucleic acid molecule encoding the mutant CPC acylase of any one of claims 1 to 5.

7. A recombinant expression vector, carrying the nucleic acid molecule of claim 6.

8. A recombinant cell, comprising the nucleic acid molecule of claim 6.

9. A use of a composition in production of 7-aminocephalosporanic acid (7-ACA) or a salt thereof, the composition comprising the mutant CPC acylase of any one of claims 1 to 5.

10. A method for producing a compound of Chemical Formula 2 or a salt thereof, the method comprising a step of contacting the composition as defined in claim 9 with a compound of Chemical Formula 1 or a salt thereof: 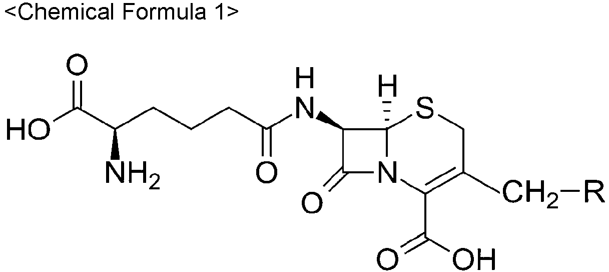 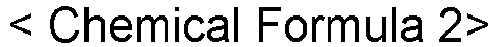 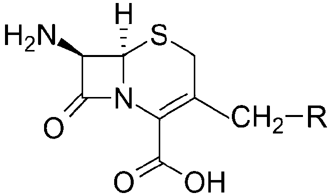 wherein, R is acetoxy(-OCOCH₃), hydroxy (-OH), or hydrogen (-H) group.

## Patentansprüche

1. Mutierte Cephalosporin-C (CPC)-Acylase, abgeleitet von einer CPC-Acylase,
wobei die mutierte CPC-Acylase eine Alpha-Untereinheit von SEQ ID NO: 3 und eine Beta-Untereinheit von SEQ ID NO: 4 sowie alle folgenden Substitutionen umfasst:
Substitution von Alanin an Position 11 in der Alpha-Untereinheit (A11α) durch Asparagin (N);
Substitution von Glycin an Position 24 in der Alpha-Untereinheit (G24α) durch Asparaginsäure (D);
Substitution von Alanin an Position 136 in der Beta-Untereinheit (A136β) durch Threonin (T); und
Substitution von Isoleucin an Position 179 in der Beta-Untereinheit (I179β) durch Tyrosin (Y).

2. Die mutierte CPC-Acylase nach Anspruch 1, wobei die mutierte CPC-Acylase ferner eine Substitution von Histidin an Position 453 in der Beta-Untereinheit von SEQ ID NO: 4 durch Threonin (T) umfasst.

3. Die mutierte CPC-Acylase nach Anspruch 1 oder 2, wobei die mutierte CPC-Acylase weiterhin eine zusätzliche Substitution mindestens einer Aminosäure umfasst, welche ausgewählt ist aus der Gruppe bestehend aus:
Substitution von I45β durch Valin (V) (I45βV);
Substitution von F58β durch Valin (V) (F58ßV);
Substitution von Y153β durch Threonin (T) (Y153ßT);
Substitution von F177β durch Leucin (L) (F177βL); und
Substitution von V382β durch Leucin (L) (V382βL),
in der Beta-Untereinheit von SEQ ID NO: 4.

4. Die mutierte CPC-Acylase nach Anspruch 3, wobei die zusätzliche Substitution alle folgenden Substitutionen umfasst:
Substitution von I45β durch Valin (V) (I45βV);
Substitution von F58β durch Valin (V) (F58ßV);
Substitution von Y153β durch Threonin (T) (Y153ßT);
Substitution von F177β durch Leucin (L) (F177βL); und
Substitution von V382β durch Leucin (L) (V382βL),
in der Beta-Untereinheit von SEQ ID NO: 4.

5. Die mutierte CPC-Acylase nach Anspruch 3, wobei die mutierte CPC-Acylase die Aminosäuresequenz von SEQ ID NO: 9 oder SEQ ID NO: 12 enthält.

6. Nukleinsäuremolekül, welches die mutierte CPC-Acylase nach einem der Ansprüche 1 bis 5 kodiert.

7. Rekombinanter Expressionsvektor, welcher das Nukleinsäuremolekül nach Anspruch 6 trägt.

8. Rekombinante Zelle, umfassend das Nukleinsäuremolekül nach Anspruch 6.

9. Verwendung einer Zusammensetzung zur Herstellung von 7-Aminocephalosporansäure (7-ACA) oder eines Salzes davon, wobei die Zusammensetzung die mutierte CPC-Acylase gemäß einem der Ansprüche 1 bis 5 umfasst.

10. Verfahren zur Herstellung einer Verbindung der chemischen Formel 2 oder eines Salzes davon, wobei das Verfahren einen Schritt umfasst, bei welchem die in Anspruch 9 definierte Zusammensetzung mit einer Verbindung der chemischen Formel 1 oder einem Salz davon in Kontakt gebracht wird: 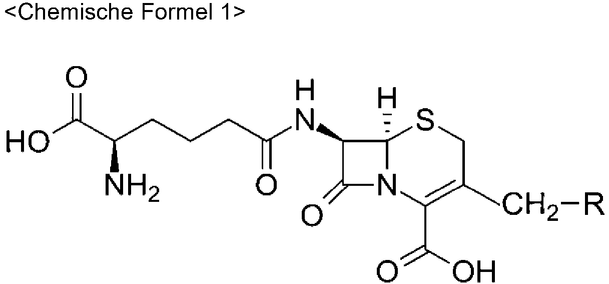 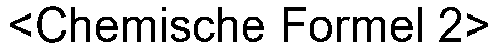 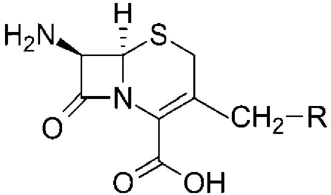 wobei R eine Acetoxygruppe (-OCOCH₃), eine Hydroxygruppe (-OH), oder ein Wasserstoffatom (-H) ist.

## Revendications

1. Acylase de la céphalosporine C (CPC) mutée, dérivée d'une acylase CPC, ladite acylase CPC mutée comprenant une sous-unité alpha de SEQ ID NO: 3 et une sous-unité bêta de SEQ ID NO: 4, ainsi que toutes les substitutions suivantes:
substitution de l'alanine en position 11 dans la sous-unité alpha (A11α) par l'asparagine (N);
substitution de la glycine en position 24 dans la sous-unité alpha (G24α) par l'acide aspartique (D);
substitution de l'alanine en position 136 dans la sous-unité bêta (A136β) par la thréonine (T); et
substitution de l'isoleucine en position 179 dans la sous-unité bêta (I179β) par la tyrosine (Y).

2. L'acylase CPC mutée selon la revendication 1, dans laquelle l'acylase CPC mutée comprend en outre une substitution de l'histidine en position 453 dans la sous-unité bêta de SEQ ID NO: 4 par de la thréonine (T).

3. L'acylase CPC mutée selon la revendication 1 ou 2, dans laquelle l'acylase CPC mutée comprend en outre une substitution supplémentaire d'au moins un acide aminé choisi dans le groupe constitué par:
la substitution de I45β par la valine (V) (I45βV);
la substitution de F58β par la valine (V) (F58βV);
la substitution de Y153β par la thréonine (T) (Y153βT);
la substitution de F177β par la leucine (L) (F177βL); et
la substitution de V382β par la leucine (L) (V382βL),
dans la sous-unité bêta de SEQ ID NO: 4.

4. L'acylase CPC mutée selon la revendication 3, dans laquelle la substitution supplémentaire comprend toutes les substitutions suivantes:
substitution de I45β par la valine (V) (I45βV);
substitution de F58β par la valine (V) (F58βV);
substitution de Y153β par la thréonine (T) (Y153βT);
substitution de F177β par la leucine (L) (F177βL); et
substitution de V382β par la leucine (L) (V382βL),
dans la sous-unité bêta de SEQ **ID** NO: 4.

5. La CPC-acylase mutée selon la revendication 3, dans laquelle la CPC-acylase mutée contient la séquence d'acides aminés de SEQ **ID** NO: 9 ou SEQ **ID** NO: 12.

6. Molécule d'acide nucléique codant pour la CPC-acylase mutée selon l'une des revendications 1 à 5.

7. Vecteur d'expression recombinant portant la molécule d'acide nucléique selon la revendication 6.

8. Cellule recombinante comprenant la molécule d'acide nucléique selon la revendication 6.

9. Utilisation d'une composition pour la préparation d'acide 7-aminocéphalosporanique (7-ACA) ou d'un sel de celui-ci, ladite composition comprenant l'acylase CPC mutée selon l'une des revendications 1 à 5.

10. Procédé de préparation d'un composé de formule chimique 2 ou d'un sel de celui-ci, ledit procédé comprenant une étape dans laquelle la composition définie dans la revendication 9 est mise en contact avec un composé de formule chimique 1 ou un sel de celui-ci : 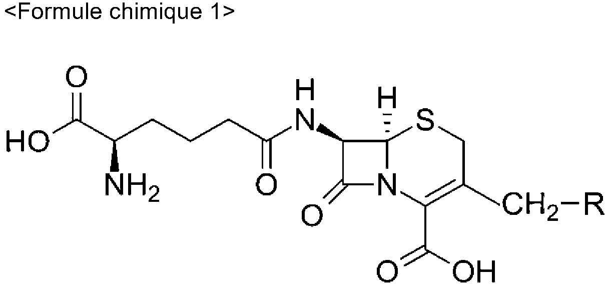 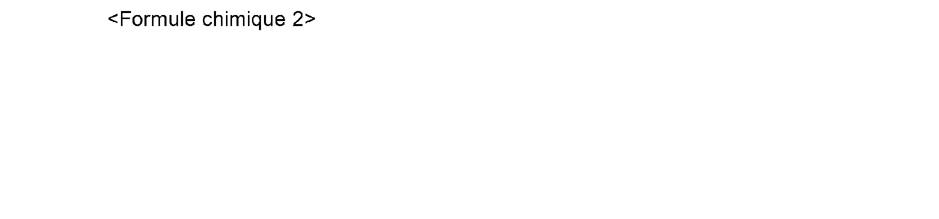 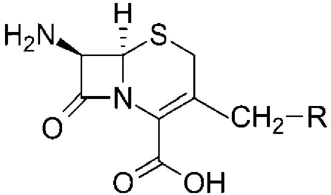 dans laquelle R représente un groupe acétoxy (-OCOCH₃), un groupe hydroxy (-OH) ou un atome d'hydrogène (-H).
